(19) Europäisches Patentamt / European Patent Office / Office européen des brevets

(11) **EP 4 250 304 B1**

(12) **EUROPEAN PATENT SPECIFICATION**

(45) Date of publication and mention
of the grant of the patent:
**04.06.2025 Bulletin 2025/23**

(51) International Patent Classification (IPC):
**G16C 20/30** (2019.01)    **G06N 3/0442** (2023.01)
**G06N 3/045** (2023.01)    **G06N 3/084** (2023.01)
**G16C 20/70** (2019.01)    **G16C 10/00** (2019.01)

(21) Application number: **23153750.7**

(22) Date of filing: **27.01.2023**

(52) Cooperative Patent Classification (CPC):
**G16C 20/30; G06N 3/0442; G06N 3/045;
G06N 3/084;** G16C 10/00; G16C 20/70

(54) **METHOD AND SYSTEM FOR DETERMINING FREE ENERGY OF PERMEATION FOR MOLECULES**

VERFAHREN UND SYSTEM ZUR BESTIMMUNG DER FREIEN PERMEATIONSENERGIE FÜR MOLEKÜLE

PROCÉDÉ ET SYSTÈME DE DÉTERMINATION DE L'ÉNERGIE LIBRE DE PERMÉATION POUR DES MOLÉCULES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **23.03.2022 IN 202221016248**

(43) Date of publication of application:
**27.09.2023 Bulletin 2023/39**

(73) Proprietor: **Tata Consultancy Services Limited
Maharashtra (IN)**

(72) Inventors:
• **DUTTA, Prantar**
 **411013 Pune, Maharashtra (IN)**
• **GUPTA, Rakesh**
 **411013 Pune, Maharashtra (IN)**
• **JAIN, DEEPAK SHYAMSUNDER**
 **411013 Pune, Maharashtra (IN)**
• **RAI, Beena**
 **411013 Pune, Maharashtra (IN)**

(74) Representative: **Goddar, Heinz J.
Boehmert & Boehmert
Anwaltspartnerschaft mbB
Pettenkoferstrasse 22
80336 München (DE)**

(56) References cited:
• **DUTTA PRANTAR ET AL: "Deep learning models for the estimation of free energy of permeation of small molecules across lipid membranes", DIGITAL DISCOVERY, vol. 2, no. 1, 21 December 2022 (2022-12-21), pages 189 - 201, XP093066791, ISSN: 2635-098X, DOI: 10.1039/ D2DD00119E**
• **BENNETT W. F. DREW ET AL: "Predicting Small Molecule Transfer Free Energies by Combining Molecular Dynamics Simulations and Deep Learning", JOURNAL OF CHEMICAL INFORMATION AND MODELING, vol. 60, no. 11, 14 August 2020 (2020-08-14), US, pages 5375 - 5381, XP093066905, ISSN: 1549-9596, Retrieved from the Internet <URL:http://pubs.acs.org/doi/ pdf/10.1021/acs.jcim.0c00318> DOI: 10.1021/ acs.jcim.0c00318**
• **BERISHVILI VLADIMIR P. ET AL: "Time-Domain Analysis of Molecular Dynamics Trajectories Using Deep Neural Networks: Application to Activity Ranking of Tankyrase Inhibitors", JOURNAL OF CHEMICAL INFORMATION AND MODELING, vol. 59, no. 8, 5 July 2019 (2019-07-05), US, pages 3519 - 3532, XP093066911, ISSN: 1549-9596, DOI: 10.1021/ acs.jcim.9b00135**

- **TSAI SUN-TING ET AL: "Learning molecular dynamics with simple language model built upon long short-term memory neural network", NATURE COMMUNICATIONS, vol. 11, no. 1, 9 October 2020 (2020-10-09), XP093066913, DOI: 10.1038/s41467-020-18959-8**

**Description**

CROSS-REFERENCE TO RELATED APPLICATIONS AND PRIORITY

The present application claims priority to Indian application no. 202221016248, filed on March 23, 2022.

TECHNICAL FIELD

The disclosure herein generally relates to molecular processing, and, more particularly, to a method and system for determining free energy of permeation of molecules across the lipid membranes.

BACKGROUND

The biological barriers such as human skin, intestine, cells, and blood brain barrier are surrounded by the lipid membranes. Permeability is an important characteristic of molecules, which determines/represents capability of the molecules to pass through these barriers. The permeability of molecules across these lipid membranes depends upon their diffusion and free energy of permeation across the lipid membranes. For example, a molecule (drug, nutritious compound) taken orally, passes through the stomach and reaches to the intestinal lipid membrane and the permeation of this active through this membrane determines its final concentrations in the systematic circulation. In another example, molecules (cosmetic ingredients) applied on the skin having a significant permeability could be used to serve intended purposes. The permeability of the molecules could be obtained using the experimental procedures using the cell lines and artificial tissues, however these methods are expensive, and requires experts and dedicated experimental facilities.

The free energy of permeation can also be determined using computational techniques such as molecular dynamics simulations and more recently using machine learning and artificial intelligence methods. State of the art techniques that are being used for determining free energy of permeation of molecules have been identified to have the following disadvantages. Estimating free energy of permeation purely based on molecular simulations is computationally expensive, and thus not suitable for high throughput calculation and screening. Machine Learning approaches suffer from low accuracy and generalizability. The free energy of permeation calculated utilizing the machine learning method only accounts for features obtained from molecules, without considering lipid membranes. Hence, the models can't capture the difference between lipids present in various biological barriers (skin, intestine, blood brain barrier and cell membranes). Document (BENNETT W. F. DREW ET AL: "Predicting Small Molecule Transfer Free Energies by Combining Molecular Dynamics Simulations and Deep Learning") discloses accurately predicting small molecule partitioning and hydrophobicity is critical in the drug discovery process. There are many heterogeneous chemical environments within a cell and entire human body. For example, drugs must be able to cross the hydrophobic cellular membrane to reach their intracellular targets, and hydrophobicity is an important driving force for drug-protein binding. Atomistic molecular dynamics (MD) simulations are routinely used to calculate free energies of small molecules binding to proteins, crossing lipid membranes, and solvation but are computationally expensive. Machine learning (ML) and empirical methods are also used throughout drug discovery but rely on experimental data, limiting the domain of applicability. We present atomistic MD simulations calculating 15,000 small molecule free energies of transfer from water to cyclohexane. This large data set is used to train ML models that predict the free energies of transfer. We show that a spatial graph neural network model achieves the highest accuracy, followed closely by a 3D-convolutional neural network, and shallow learning based on the chemical fingerprint is significantly less accurate. A mean absolute error of ~4 kJ/mol compared to the MD calculations was achieved for our best ML model. We also show that including data from the MD simulation improves the predictions, tests the transferability of each model to a diverse set of molecules, and show multitask learning improves the predictions. This work provides insight into the hydrophobicity of small molecules and ML cheminformatics modeling, and our data set will be useful for designing and testing future ML cheminformatics methods (Abstract). Document (BERISHVILI VLADIMIR P. ET AL: "Time-Domain Analysis of Molecular Dynamics Trajectories Using Deep Neural Networks: Application to Activity Ranking of Tankyrase Inhibitors") discloses that Molecular dynamics simulations provide valuable insights into the behavior of molecular systems. Extending the recent trend of using machine learning techniques to predict physicochemical properties from molecular dynamics data, we propose to consider the trajectories as multidimensional time series represented by 2D tensors containing the ligand-protein interaction descriptor values for each time step. Similar in structure to the time series encountered in modern approaches for signal, speech, and natural language processing, these time series can be directly analyzed using long short-term memory (LSTM) recurrent neural networks or convolutional neural networks (CNNs). The predictive regression models for the ligand-protein affinity were built for a subset of the PDBbind v.2017 database and applied to inhibitors of tankyrase, an enzyme of the poly(ADP-ribose)-polymerase (PARP) family that can be used in the treatment of colorectal cancer. As an additional test set, a subset of the Community Structure-Activity Resource (CSAR) data set was used. For comparison, the random forest and simple neural network models based on the crystal pose or the trajectory-averaged descriptors were used, as well as the commonly employed docking and molecular mechanics Poisson-Boltzmann surface area (MM-PBSA) scores. Convolutional neural networks based on the 2D tensors of ligand-protein interaction descriptors for short (2 ns) trajectories provide the best accuracy and predictive power, reaching the Spearman rank correlation coefficient of 0.73 and Pearson correlation coefficient of 0.70 for the tankyrase test set. Taking into account the recent increase in computational power of modern GPUs and relatively low computational complexity of the proposed approach, it can be used as an advanced virtual screening filter for compound prioritization (Abstract). Document (TSAI SUN-TING ET AL: "Learning molecular dynamics with simple language model built upon long short-term memory neural network") discloses Recurrent neural networks have led to breakthroughs in natural language processing and speech recognition. Here we show that recurrent networks, specifically long short-term memory networks can also capture the temporal evolution of chemical/biophysical trajectories. Our character-level language model learns a probabilistic model of 1-dimensional stochastic trajectories generated from higher-dimensional dynamics. The model captures Boltzmann statistics and also reproduces kinetics across a spectrum of timescales. We demonstrate how training the long short-term memory network is equivalent to learning a path entropy, and that its embedding layer, instead of representing contextual meaning of characters, here exhibits a nontrivial connectivity between different metastable states in the underlying physical system. We demonstrate our model's reliability through different benchmark systems and a force spectroscopy trajectory for multi-state riboswitch. We anticipate that our work represents a stepping stone in the understanding and use of recurrent neural networks for understanding the dynamics of complex stochastic molecular systems (Abstract).

SUMMARY

The invention is defined by the claims.

Embodiments of the present disclosure present technological improvements as solutions to one or more of the above-mentioned technical problems recognized by the inventors in conventional systems. For example, in one embodiment, a processor implemented method is provided. In this method, initially a first set of features is generated via one or more hardware processors, based on interaction of a molecule with a lipid membrane, by performing a molecular dynamics simulation, wherein values of the first set of features are collected as a time series data. Further, a second set of features is generated via the one or more hardware processors, based on interaction of the molecule with a solvent, by performing the plurality of molecular dynamics simulations, wherein values of the second set of features are collected as a time series data. Further, a concatenated data set comprising values of the first set of features and the second set of features is generated, via the one or more hardware processors, wherein the values of the first set of features and the second set of features are extracted from a plurality of instances of the time series data of the first set of features and the second set of features. Further, the concatenated data set is pre-processed via the one or more hardware processors to generate a pre-processed data set. Further, the pre-processed data set is represented in a 2-Dimensional (2D) array format via one or more hardware processors. Further, a free energy of permeation is generated via the one or more hardware processors, by processing the pre-processed data set in the 2D array format using a machine learning data model. Processing the pre-processed data set in the 2D array format using the machine learning data model includes the following steps. A context vector comprising information on time dependency, feature relevance, and similarity, for a plurality of molecule-lipid membrane combinations in the pre-processed data set stored in the 2D array format, is generated. Further, a transformed vector comprising information on non-linear dependency of the components of the context vector, is generated. Further, the free energy of permeation is generated as weighted sum of components of the transformed vector.

In another aspect, a system is provided. The system includes one or more hardware processors, a communication interface, and a memory (104) storing a plurality of instructions, wherein the plurality of instructions when executed, cause the one or more hardware processors to initially generate a first set of features, based on interaction of a molecule with a lipid membrane, by performing a plurality of molecular dynamics simulations, wherein values of the first set of features are collected as a time series data. Further, the system generates a second set of features via the one or more hardware processors, based on interaction of the molecule with a solvent, by performing the plurality of molecular dynamics simulations, wherein values of the second set of features are collected as a time series data. Further, a concatenated data set comprising values of the first set of features and the second set of features is generated, via the one or more hardware processors, wherein the values of the first set of features and the second set of features are extracted from a plurality of instances of the time series data of the first set of features and the second set of features. Further, the concatenated data set is pre-processed via the one or more hardware processors to generate a pre-processed data set. Further, the pre-processed data set is represented in a 2-Dimensional (2D) array format via one or more hardware processors. Further, a free energy of permeation is generated via the one or more hardware processors, by processing the pre-processed data set in the 2D array format using a machine learning data model. Processing the pre-processed data set in the 2D array format using the machine learning data model includes the following steps. A context vector comprising information on time dependency, feature relevance, and similarity, for a plurality of molecule-lipid membrane combinations in the pre-processed data set stored in the 2D array format, is generated. Further, a transformed vector comprising information on non-linear dependency of the components of the context vector, is generated. Further, the free energy of permeation is generated as weighted sum of components of the transformed vector.

In yet another aspect, a non-transitory computer readable medium is provided. A plurality of instructions in the non-transitory computer readable medium when executed, cause one or more hardware processors to perform the following steps. Initially, a first set of features is generated via the one or more hardware processors, based on interaction of a molecule with a lipid membrane, by performing a plurality of molecular dynamics simulations, wherein values of the first set of features are collected as a time series data. Further, a second set of features is generated via the one or more hardware processors, based on interaction of the molecule with a solvent, by performing the plurality of molecular dynamics simulations, wherein values of the second set of features are collected as a time series data. Further, a concatenated data set comprising values of the first set of features and the second set of features is generated, via the one or more hardware processors, wherein the values of the first set of features and the second set of features are extracted from a plurality of instances of the time series data of the first set of features and the second set of features. Further, the concatenated data set is pre-processed via the one or more hardware processors to generate a pre-processed data set. Further, the pre-processed data set is represented in a 2-Dimensional (2D) array format via one or more hardware processors. Further, a free energy of permeation is generated via the one or more hardware processors, by processing the pre-processed data set in the 2D array format using a machine learning data model. Processing the pre-processed data set in the 2D array format using the machine learning data model includes the following steps. A context vector comprising information on time dependency, feature relevance, and similarity, for a plurality of molecule-lipid membrane combinations in the pre-processed data set stored in the 2D array format, is generated. Further, a transformed vector comprising information on non-linear dependency of the components of the context vector, is generated. Further, the free energy of permeation is generated as weighted sum of components of the transformed vector.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary system for determining free energy of permeation, according to some embodiments of the present disclosure.

FIGS. 2A and 2B (collectively referred to as FIG. 2) is a flow diagram depicting steps involved in the process of generating the free energy of permeation, using the system of FIG. 1, according to some embodiments of the present disclosure.

FIG. 3 is a flow diagram depicting steps involved in the process of extracting values of a first set of features and a second set of features, using the system of FIG. 1, in accordance with some embodiments of the present disclosure.

FIGS. 4A and 4B depict examples of molecule in lipid membrane system (bi-layer) and molecule in solvent system, in accordance with some embodiments of the present disclosure.

FIG. 5 is an example of a neural network architecture used for determining the free energy of permeation by the system of FIG. 1, in accordance with some embodiments of the present disclosure.

FIG. 6 is an example architecture of LSTM layer of the neural network of FIG. 5, in accordance with some embodiments of the present disclosure.

FIG. 7 is an example architecture of an attention layer of the neural network of FIG. 5, in accordance with some embodiments of the present disclosure.

FIGS. 8A, 8B, and 8C (collectively referred to as FIG. 8) depict plots of values of free energy of permeation obtained for different datasets, in an experimental setup of the system of FIG. 1, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Permeability is an important characteristic of molecules, which determines/represents capability of the molecules to pass through various membranes. For example, molecules of a drug, when ingested to a human body, may have to pass through various membranes in the body to reach a target cell. In another example, various molecules in cosmetic products also are required to have specific permeability values to serve intended purposes. Traditional systems estimating free energy of permeation purely based on molecular simulations are computationally expensive, and thus not suitable for high throughput calculation and screening. Existing machine learning approaches suffer from low accuracy and generalizability. The free energy of permeation is based on the molecule only, without considering lipid membranes. Hence, the models can't capture the difference between lipids.

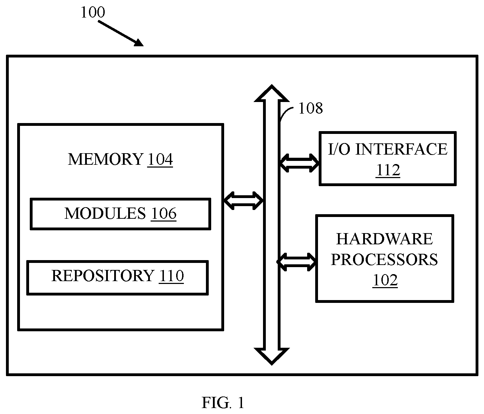

Referring now to the drawings, and more particularly to FIG. 1 through FIG. 8C, where similar reference characters denote corresponding features consistently throughout the figures, there are shown preferred embodiments and these embodiments are described in the context of the following exemplary system and/or method.

FIG. 1 illustrates an exemplary system for determining free energy of permeation, according to some embodiments of the present disclosure. The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in the memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106.

The plurality of modules 106 include programs or coded instructions that supplement applications or functions performed by the system 100 for executing different steps involved in the process of determining the free energy of permeability of molecules, being performed by the system 100. The plurality of modules 106, amongst other things, can include routines, programs, objects, components, and data structures, which performs particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for the spike data optimization.

The data repository (or repository) 110 may include a plurality of abstracted piece of code for refinement and data that is processed, received, or generated as a result of the execution of the plurality of modules in the module(s) 106.

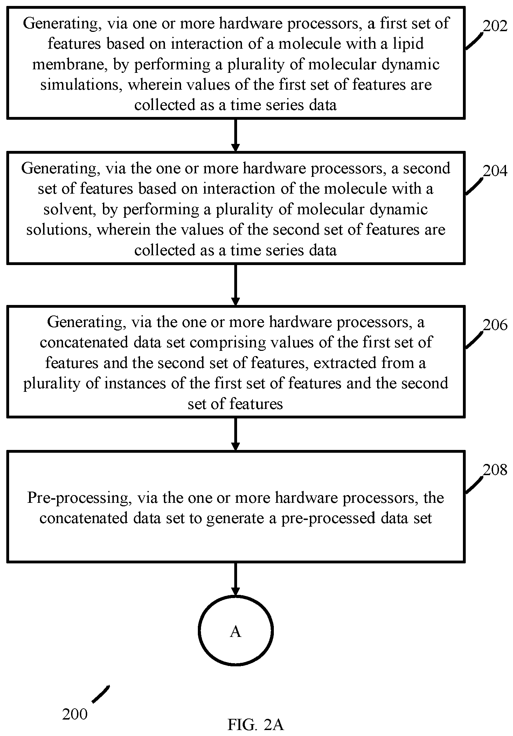

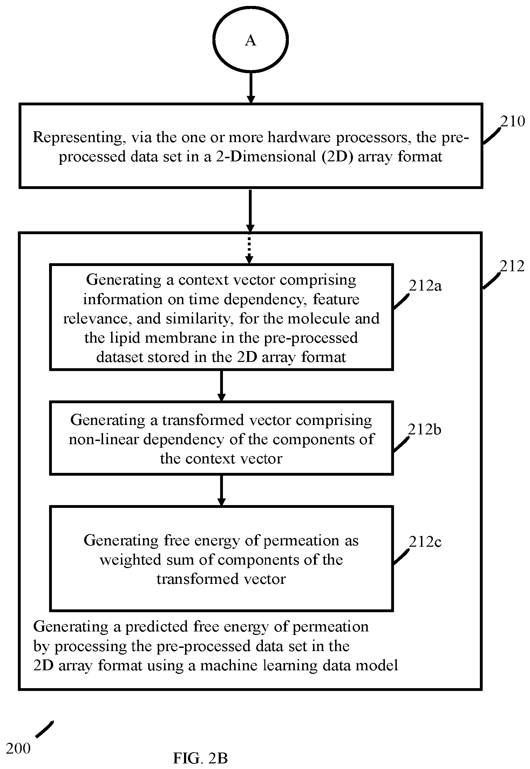

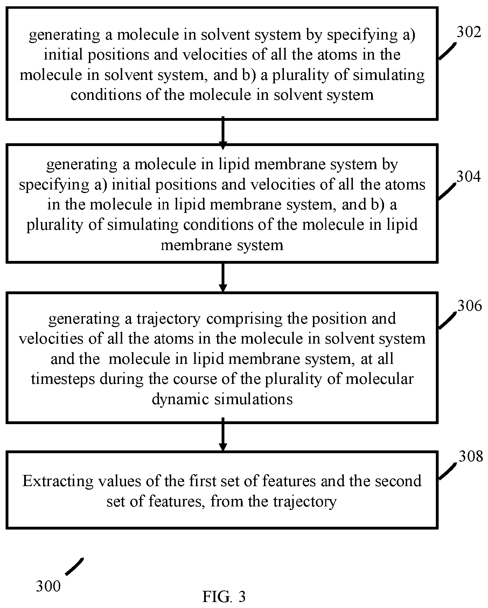

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 100 are now explained with reference to steps in flow diagrams in FIG. 2 and FIG. 3.

FIGS. 2A and 2B (collectively referred to as FIG. 2) is a flow diagram depicting steps involved in the process of determining the free energy of permeation, using the system of FIG. 1, according to some embodiments of the present disclosure.

At step 202 of a method 200 in FIG. 2, the one or more hardware processors 102 of the system 100 are configured to generate a first set of features based on interaction of a molecule with a lipid membrane, by performing a plurality of molecular dynamic simulations, wherein values of the first set of features are collected as a time series data. The first set of features comprises a) interaction energy (Lennard-Jones (LJ)) of the molecule with the lipid membrane, b) area per lipid of the lipid membrane, c) root mean square (RMS) deviation of a plurality of lipid molecules from a corresponding initial position, and d) RMS deviation for combination of a plurality of lipid membrane and the molecule.

Similarly, at step 204 of the method 200, the one or more hardware processors 102 of the system 100 are configured to generate a second set of features based on interaction of a molecule with a solvent, by performing the plurality of molecular dynamic simulations, wherein values of the second set of features are collected as a time series data. The second set of features comprises a) surface area of the molecule that can be accessed by the solvent, b) molecule-solvent enthalpy, c) LJ interaction energy between the molecule and the solvent, and d) bond energy of the molecule.

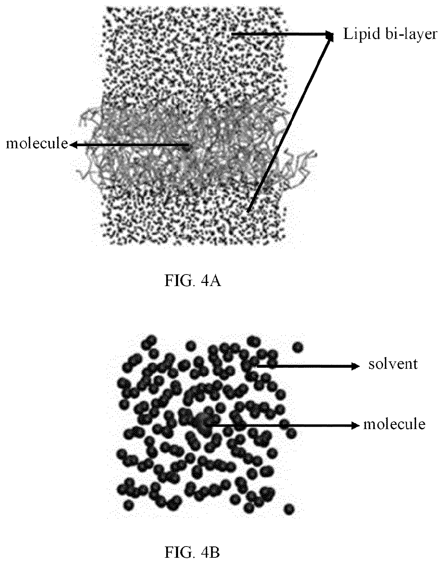

The steps 202 and 204 are further explained with reference to FIG. 3. In order to observe the interaction of one molecule with the solvent, at step 302 of method 300 in FIG. 3, the molecule in solvent system is generated by creating a simulation box and inserting the molecule and solvent molecules in the simulation box, wherein insertion refers to specifying the initial positions and velocities of all atoms in the system. For example, the molecule can be inserted at the center of the simulation box and the remaining box volume is filled up with an appropriate number of solvent molecules. In a similar manner, the molecule in lipid membrane system also is generated at step 304, by creating a simulation box and specifying initial positions and velocities of all atoms of the molecule and lipid molecules. For example, lipid molecules can be arranged in the form of a membrane of at least one type of lipid and the molecule can be inserted at the membrane midplane. Examples of molecule in lipid membrane system (bi-layer) and molecule in solvent system, are depicted in FIG. 4A and FIG. 4B respectively.

In an embodiment, selection of the solvent may be based on requirements. For example, the solvent may be water, or ethanol, or a combination thereof, in various concentration ratio. Similarly, the lipid membrane may be appropriately selected based on requirements. Examples of the membranes are, but not limited to skin's stratum corneum, intestine, and cell membrane.

After creating the molecule in solvent system and the molecule in lipid membrane system, the simulation conditions are specified. For example, the simulations can be performed at room temperature (300 K) or physiological temperature (310 K), and atmospheric pressure, for pharmaceutical applications. The initial molecule in solvent system and molecule in lipid membrane system are subjected to potential energy minimization, wherein any overlaps between one or more pairs of atoms are removed..

At each instance, value of each of the features forming the first set of features and the second set of features is obtained/calculated by the system 100. Further, at step 306, the system 100 generates a trajectory comprising position and velocities of atoms in the molecule in solvent system and the molecule in lipid membrane system, at all timesteps during the course of the plurality of molecular dynamic simulations.. The molecular dynamics simulations are performed for the molecule in solvent system and the molecule in lipid system to obtain the trajectory, wherein the trajectory refer to or includes the positions and velocities of all the atoms at all time steps during the course of the plurality of molecular dynamic simulation. The plurality of features forming the first set of features and the plurality of features forming the second set of features are calculated/extracted from these trajectories at step 308 of the method 300. In the trajectory, each single configuration is referred to as a snapshot, which consists of the positions and velocities of all particles at a particular time instant. Values of each of the features forming the first set and the second set of features is calculated as a function of the positions and velocities of the particles. Thus, from the trajectory, multiple snapshots can be extracted, and accordingly the values of the features may be calculated.

Each of the features forming the first set of features and the second set of features is defined below:

1. Bondener (calculated from molecule-solvent simulation):-
For each bond in the molecule, there is a corresponding bond energy. This value depends on (or is a complex mathematical function of) an instantaneous bond length during a simulation, equilibrium bond length, and bond constant. The equilibrium bond length, and bond constant are specified as a part of the interatomic interactions while creating molecular models. As the coordinates of all molecules are known, the distance between two bonded particles is calculated as the instantaneous bond length. The bond energies of all individual bonds of the drug molecules are added and the sum is divided by the total number of bonds to compute the 'bondener' feature.

2. Lj-mol-wat (calculated from molecule-solvent simulation):-
Lennard-Jones (LJ) interactions are non-bonded interactions between two particles. It depends on the nature of the individual particles (there are two parameters $\sigma$ and $\varepsilon$ that defines the particles) and their interparticle distance. The LJ interaction between the molecule and molecules of the solvent is calculated because the coordinates are known for each instance. All the individual interaction values are added, and the sum is divided by the number of molecules.

3. Molwat-enthalpy (calculated from molecule-solvent simulation):-
Complex mathematical function of the coordinates of all particles in the molecule-solvent simulation.

4. Sasa (calculated from molecule-solvent simulation):-
Surface area of the molecule that can be accessed by the solvent. It is also a complex mathematical function of coordinates.

5. Lj-mol-lip (calculated from molecule-lipid membrane simulation):-

Similar to lj-mol-wat, but interactions are calculated between the molecule and lipid membrane.

6. Apl (calculated from molecule-lipid membrane simulation):-

Apl is area per lipid. The lipids membrane exists in the form of bilayers consisting of two leaflets (top and bottom). The Apl is calculated as the cross-sectional area of the simulation box divided by the number of lipids in each leaflet.

7. Rmsd-lip (calculated from molecule-lipid membrane simulation):-

Root mean square deviation of the lipid particles from initial position. It can be calculated from the coordinates of a plurality of lipid membrane particles at an instance.

8. Rmsd-mollip (calculated from molecule-lipid membrane simulation):-

Similar to rmsd-lip, but combination of lipid membrane and molecules is considered.

After generating the first set of features and the second set of features using the method 300 of FIG. 3, further at step 206 of the method 200, the system 100 generates, via the one or more hardware processors, a concatenated data set comprising values of the first set of features and the second set of features, extracted from a plurality of instances of the first set of features and the second set of features. Further, at step 208 of the method 200, the system 100 pre-processes, via the one or more hardware processors, the concatenated data set to generate a pre-processed data set. Pre-processing the dataset comprises steps such as but not limited to data scaling. The system 100 may use any suitable technique for the data scaling. In another embodiment, in addition to data scaling, the system 100 may perform any other required actions so as to condition the data in the concatenated data set as required for further processing. For example, the system 100 may change formatting of the data to match a required format, as part of the pre-processing.

Further, at step 210 of the method 200, the system 100 represents, via the one or more hardware processors, the pre-processed data set in a 2-Dimensional (2D) array format. The 2D array format allows representing the time-series data with multiple features. As compared to one-dimensional (1D) static/average features, the 2D array format allows feeding comparatively larger amount of data.

Further, at step 212 of the method 200, the system 100 generates a free energy of permeation by processing the pre-processed data set in the 2D array format using a machine learning data model. The machine learning data model is generated by using data obtained for a plurality of molecule-lipid layer combinations i.e. different molecules and different lipid layers, so that the machine learning data model can be used for determining free energy of permeation for different molecule-lipid layer combinations received as input. Processing the pre-processed data set in the 2D array format using a machine learning data model includes the following sub-steps. At sub-step 212A, the system 100 generates a context vector comprising information on time dependency, feature relevance, and similarity, for a plurality of molecules in the pre-processed dataset stored in the 2D array format. The 2D array (alternately referred to as 'array') also represents the evolution of the molecule in solvent system and/or the molecule in lipid membrane system with time. Consider that the first row of the array represents the features of the molecule in solvent system and/or the molecule in lipid membrane system at a particular time instant, the second row represents the features of the molecule in solvent system and/or the molecule in lipid membrane system at a later time instant, and so on (as given in Table. 1). Hence, by moving across the rows, information on how the features of the molecule in solvent system and/or the molecule in lipid membrane system changes with time is obtained. This is referred to as the time dependency. Similarly, as change in values of features across different time instances is a crucial data, it is important that the values of same features are monitored and calculated across instances, and is referred to as the feature dependency. The feature relevance means that various model weights used while building the data model are adjusted during a training process in such a way that the output is sensitive to some features more than others. Higher relevance of a feature indicates a stronger correlation between a feature and the output. The training process of any machine learning model involves tuning the weights and biases using an optimization approach, so that the output accuracy is maximized. The data model learns the relative importance of each feature during training. The context vector is calculated using an attention layer of the data model, and captures similarity between different molecules. For example, the context vectors of two hydrophobic molecules (negative free energy of permeation) are closer to each other in comparison context vectors of molecules having hydrophobic and a hydrophilic nature. The context vector also represents a relative importance of the features for computing the free energy of permeation. The context vector also reduces a sequential information into a simple vector that can be mapped to output of the data model i.e. the free energy of permeation.

At sub-step 212b, the system 100 generates a transformed vector comprising information on non-linear dependency of the components of the context vector. Further, at sub-step 212c, the system 100 determines the free energy of permeation as weighted sum of components of the transformed vector.

The free energy of permeation may be then provided as a recommendation to the user, by the system 100. In an embodiment, the free energy of permeation may be displayed to the user using an appropriate display interface of the system 100 or any other user device (for example, a mobile phone, a tablet PC and so on) that is configured to communicate with the system 100.

Experimental Data:

Values of the features forming the first feature set and the second feature set were obtained by conducting the molecular dynamics simulations, for a selected drug-lipid membrane combination, are represented by a 2D array of time series features, such that there are 8 columns (no. of features) and 350 rows (no. of time steps) for every sample, as given below:

|  | Feat 1 | Feat 2 | Feat 3 | Feat 4 | Feat 5 | Feat 6 | Feat 7 | Feat 8 |
|---|---|---|---|---|---|---|---|---|
| Step 1 | 2.960 | -37.671 | -4874.3 | 0.801 | -39.273 | 0.587 | 1.908 | 1.907 |
| Step 2 | 17.12 | -42.761 | -4944.4 | 0.704 | -37.091 | 0.599 | 1.833 | 1.831 |

. . . . . .
.
. . . . . .

|  | | | | | | | | |
|---|---|---|---|---|---|---|---|---|
| Step 350 | 2.441 | -39.602 | -4925.7 | 0.818 | -34.785 | 0.589 | 3.316 | 3.313 |

Table. 1

Where,

- Feat 1 (Feature 1) is Bondener :- contributes to the energetics
- Feat 2 (Feature 2) is Lj-mol-wat - contributes to the energetics
- Feat 3 (Feature 3) is Molwat-enthalpy - contributes to the energetics
- Feat 4 (Feature 4) is Sasa - related to hydrophobic effect
- Feat 5 (Feature 5) is Lj-mol-lip - contributes to the energetics
- Feat 6 (Feature 6) is Apl - determines how much space the lipid bilayer allows to facilitate the entry of drug; indirectly related to entropy
- Feat 7 (Feature 7) is Rmsd-lip - related to entropy, determines if the lipid molecules are mobile enough to readjust their configuration
- Feat 8 (Feature 8) is Rmsd-mollip- related to entropy, determines the combined mobility of lipids and drug

The data, which is a raw input, is scaled using a Min-Max scaler, and the scaled input matrix is fed to the data model. The scaled input of the above sample is given as follows (in Table. 2):

|  | Feat 1 | Feat 2 | Feat 3 | Feat 4 | Feat 5 | Feat 6 | Feat 7 | Feat 8 |
|---|---|---|---|---|---|---|---|---|
| Step 1 | 0.0945 | 0.5097 | 0.4793 | 0.4717 | 0.3315 | 0.0899 | 0.3273 | 0.3267 |
| Step 2 | 0.5469 | 0.4385 | 0.3763 | 0.2915 | 0.3670 | 0.1209 | 0.3074 | 0.3068 |

. . . . . .

.

. . . . . .

.

| Step 350 | 0.0780 | 0.4827 | 0.4037 | 0.5042 | 0.4045 | 0.0962 | 0.6994 | 0.6980 |
|---|---|---|---|---|---|---|---|---|

Table. 2

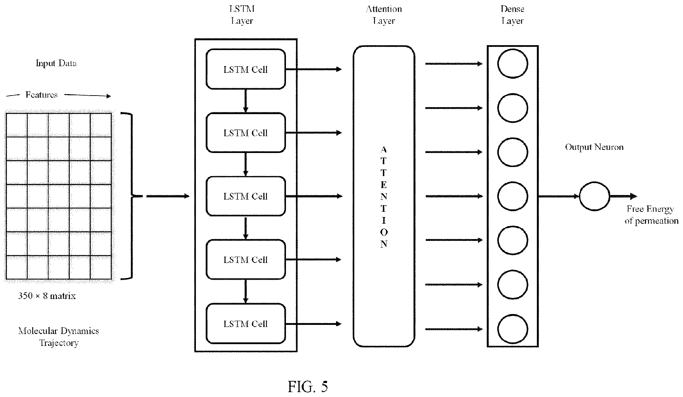

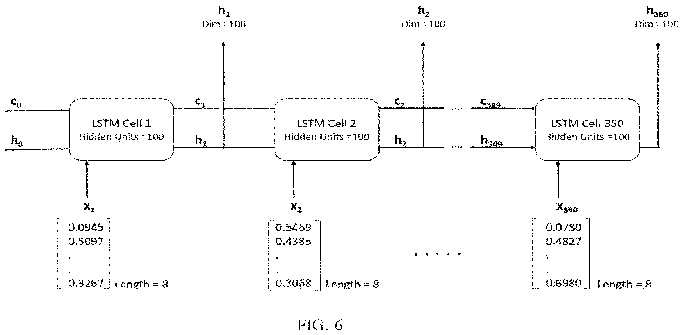

This data is further used to train the data model comprising the LSTM layers and the attention layer of a neural network. Example architecture of the neural network is depicted in FIG. 5. Further, example architecture of the LSTM layer of the neural network of FIG. 5 is depicted in FIG. 6. As depicted in FIG. 6, the LSTM layer encodes the physically-relevant features into computational states for further processing. The LSTM layer uses feature vectors $(x_1, x_2, ..., x_{350})$ of length 8 corresponding to Step 1, Step 2, ...., Step 350. Each LSTM cell of the LSTM layer takes the feature vector at each time step $(x_t)$, hidden state of the previous cell $(h_{t-1})$, and the previous cell state $(c_{t-1})$ as input, and produces the cell state $(c_t)$ and hidden state $(h_t)$ as output. The hidden states $h_1, h_2, ..., h_{350}$ are of length 100 (corresponding to 100 hidden units), and they are obtained by a series of mathematical transformations of the inputs data. From the LSTM layer, an output of 350 hidden states of length 100 were obtained as output which is then fed as input to the attention layer.

Layer weights that determine the hidden states were optimized during training of the data model (i.e. training phase) such that the time dependency of the features is preserved. As free energy depends on the distribution of possible states of the molecule in solvent system and/or the molecule in lipid membrane system, how states evolve with time and identify the relevant states attained during a process is to be considered, both of which are accomplished by the LSTM layer. Thus, the LSTM layer is a way of encoding the input so that subsequent layers can learn relevant information from the hidden states.

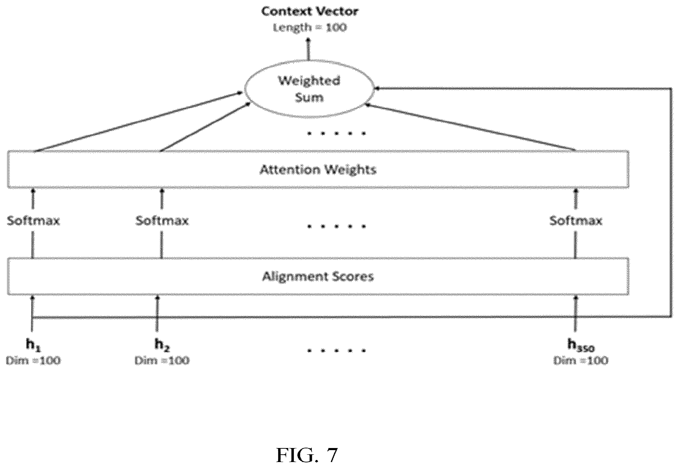

The hidden states from the LSTM layer are fed into the Attention layer to generate the context vector. An example architecture of the attention layer of the neural network of FIG. 5 is depicted in FIG. 7. The context vector captures similarity between different molecules. For example, the context vectors of two hydrophobic molecules (negative free energy of permeation) are closer to each other in comparison context vectors of molecules having hydrophobic and a hydrophilic nature. The context vector also represents a relative importance of the features for computing the free energy of permeation. The context vector also reduces a sequential information into a simple vector that can be mapped to output of the data model i.e. the free energy of permeation.

From the encoded hidden states of the previous LSTM layers, a plurality of alignment scores were calculated using a single-layered feedforward network by the attention layer. Then, the attention weights were computed by applying a SoftMax activation function on the alignment scores. Finally, a weighted sum over all input time steps is performed to generate the context vector. The relevant equations are as follows:

$$a = \tanh(W^a h + b^a) \qquad \rightarrow (1)$$

where 'a' is the alignment score matrix, h is the matrix of hidden states from the LSTM layer, $W^a$ and $b^a$ are the weights and biases of the feedforward network, and 'tanh' is the activation function.

$$\alpha = SoftMax(a) \qquad \rightarrow (2)$$

where $\alpha$ is the attention weight matrix.

$$C^o = \Sigma \, \alpha \cdot h \qquad \rightarrow (3)$$

where C° is the context vector of length 100, calculated as the weighted sum from the hidden states. The LSTM layer and the attention layer were sequentially used to learn the time dependency, the feature relevance, and the similarity between molecules.

Further, a dense layer and an output neuron mapped the context vector to the free energy of permeation through a series of mathematical transformations. The context vector encoded all the relevant information required for estimating the free energy of permeation, and the dense layer learns the non-linear dependence of the elements of the context vector with the free energy. Hence, by passing the context vector through the dense layer, another vector of same length that incorporates the nonlinearity, was obtained, and is represented as:

$$D = ReLU(W^d \cdot C^o + b^d) \qquad \rightarrow (4)$$

where, $W^d$ and $b^d$ are the weight and bias matrices of the dense layer, C° is the context vector from the attention layer, D is the output of the dense layer, and ReLU is the activation function. Similar to the context vector, the output of the dense layer was also of length 100.

Then the free energy of permeation was estimated in the output neuron as the weighted sum of the elements of the transformed vector of the dense layer.

$$Free\ Energy\ of\ Permeation = w_1 \cdot D_1 + w_2 \cdot D_2 + \ldots + w_{100} \cdot D_{100} + b_{output} \qquad \rightarrow (5)$$

where, $D_1, D_2, ..., D_{100}$ are the elements of the vector D (output of the dense layer); $w_1, w_2, ...., w_{100}$ are the weights of the output neuron, and $b_{output}$ is the bias.

While estimating the free energy of permeation, a Mean Absolute Error (MAE) loss function was used to train the data model, which is a standard practice for regression problems. During the training phase, the deviations of the free energy values calculated by the model from the actual values were backpropagated, and the weights of all the layers were adjusted such that this deviation is minimized. After the training phase, the trained data model then used for processing real-time inputs and for generating value of the free energy of permeation corresponding to values of the real-time inputs.

Results: -

For the experiments, the drug-lipid membrane combinations in the training, validation, and test datasets were 510, 57, and 63. The machine learning model was additionally validated on an out-of-distribution dataset of 50 drug-lipid combinations. The determined values of free energy of permeation (alternately referred to as 'prediction' or 'free energy estimation' while explaining the experimental results) were compared against actual free energy values computed using state of the art approaches. For the data model to be reliable for high-throughput free energy estimation, statistical error must be within tolerable limits. Due to the natural thermal fluctuations in MD simulation-based methods, an error around 1-2 $k_B T$ (~ 0.6-1.2 kcal/mol at 300 K) can be considered extremely accurate. The model was trained for 1000 epochs to reduce the validation loss, and the MAE and the coefficient of determination ($R^2$) was reported to evaluate model performance.

Table 3: Mean Absolute Error (MAE) (in kcal/mol), and Coefficient of Determination ($R^2$) of the deep learning model on the training, validation, test, and out-of-distribution sets:

| Training Set | | Validation Set | | Test Set | | Out-of-dist Set | |
|---|---|---|---|---|---|---|---|
| MAE | $R^2$ | MAE | $R^2$ | MAE | $R^2$ | MAE | $R^2$ |
| 0.24 | 0.996 | 0.34 | 0.992 | 0.46 | 0.984 | 1.50 | 0.82 |

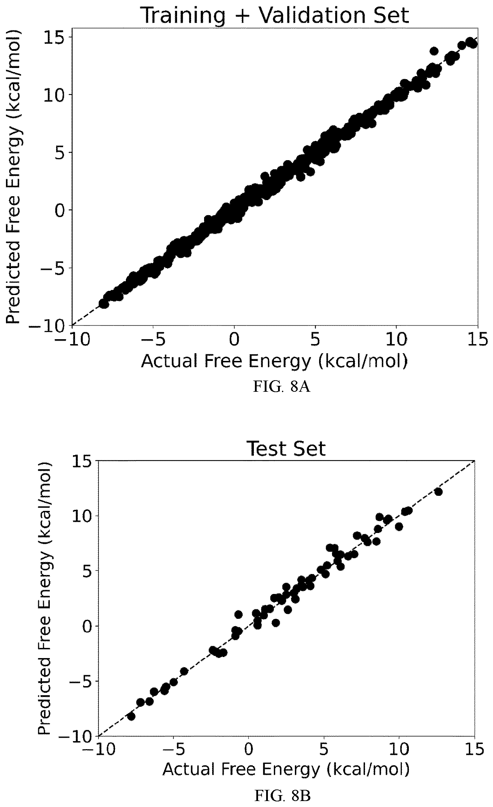

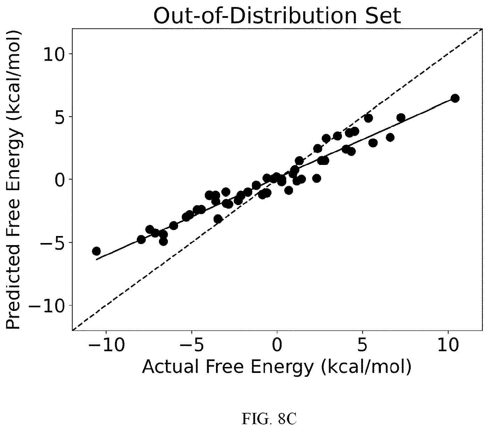

Table 3 summarizes the performance metrics of the model on the training, validation, test, and out-of-distribution sets. The results show that the model is powerful in predicting the free energy of permeation from the multidimensional time series generated using MD simulations. The MAEs on the training, validation, and test datasets are within acceptable limits and lower than state-of-the-art data-driven methods, with $R^2$ being around 0.99. The comparable results in all three cases indicate the generalizability of the data model being used by system 100. The prediction accuracy for the out-of-distribution dataset is lower than the test set due to the difference in drug chemistry, but it still outperforms state-of-the-art data-driven methods. FIGS. 8A, 8B, and 8C plot the determined free energies with the actual free energies from the dataset.

It is vital in virtual screening applications to correctly rank molecules based on their relative free energy of permeation even if the absolute predictions are not too accurate. Generally, a few top candidates are chosen from hundreds or thousands after screening and subjected to experiments. The Pearson correlation coefficient, which measures the linear correlation between two variables, and Spearman's rank correlation coefficient, which measures the extent of monotonicity between two variables, for the model were calculated to be 0.969 and 0.967, respectively, on the out-of-distribution set. Hence, the data model can be reliably deployed to screen small drug-like molecules. The system was designed to devise a solution to estimate the free energy of permeation of small molecules across membranes with similar accuracy as MD simulation-based methods but at a much faster rate. As most virtual screening tasks involve hundreds or thousands of molecules, the computational paradigm must be well equipped to handle them within a realistic time frame. The system and method in the embodiments disclosed herein were benchmarked on a single AMD Ryzen 5 3500U processor with 4 GB RAM. The drug-water simulation of each system, including equilibration, took around 6.5 minutes, whereas 105 minutes were required for drug-lipid membrane simulations. The approximate time required for estimating free energy of permeation for each drug-lipid combination on the same computer using state-of-the-art purely molecular simulation techniques was found to be 45 to 55 hours. Comparing results of the model of the system 100 with a state of the art system, it was observed that a speed-up of about 25x can be achieved by following our system and method. Similar speed-ups are expected for other system architectures like GPUs and clusters.

The written description describes the subject matter herein to enable any person skilled in the art to make and use the embodiments..

The embodiments of present disclosure herein address unresolved problem of determining free energy of permeation. The embodiment, thus provides a method and system for determining a free energy of permeation for molecules. Moreover, the embodiments herein further provides a mechanism to use features generated based on interaction of molecules with solvents and lipid membrane together to determine the free energy of permeation.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1.   A processor implemented method (200), comprising:

generating (202), via one or more hardware processors, a first set of features based on interaction of a molecule with a lipid membrane, by performing a plurality of molecular dynamics simulations, wherein values of the first set of features are collected as a time series data, wherein the first set of features comprises a) interaction energy Lennard-Jones, LJ, of the molecule with the lipid membrane, b) area per lipid of the lipid membrane, c) root mean square (RMS) deviation of a plurality of lipid molecules from a corresponding initial position, and d) RMS deviation for combination of a plurality of lipid membrane and the molecule;

generating (204), via the one or more hardware processors, a second set of features based on interaction of the molecule with a solvent by performing the plurality of molecular dynamics simulations, wherein values of the second set of features are collected as a time series data, wherein the second set of features comprises a) surface area of the molecule that can be accessed by the solvent, b) molecule-solvent enthalpy, c) LJ interaction energy between the molecule and the solvent, and d) bond energy of the molecule;

generating (206), via the one or more hardware processors, a concatenated data set comprising values of the first set of features and the second set of features, extracted from a plurality of instances of the time series data of the first set of features and the second set of features; pre-processing (208), via the one or more hardware processors, the concatenated data set to generate a pre-processed data set, wherein generating the first set of features and the second set of features comprises:

generating (302) a molecule in a solvent system by specifying a) initial positions and velocities of all the atoms in the molecule in the solvent system, and b) a plurality of simulating conditions of the molecule in the solvent system, wherein simulations are performed at room temperature, 300K, or physiological temperature, 310K, atmospheric pressure, for pharmaceutical application, wherein the molecule in the solvent system is generated by creating a simulation box and inserting the molecule and solvent molecules in the simulation box, wherein inserting refers to specifying initial positions and velocities of all the atoms in the molecule in the solvent system and the molecule is inserted at the center of the simulation box and remaining box volume is filled up with solvent molecules;

generating (304) a molecule in a lipid membrane system by specifying a) initial positions and velocities of all the atoms in the molecule in the lipid membrane system, and b) a plurality of simulating conditions of the molecule in the lipid membrane system, wherein the molecule in the lipid membrane system is generated by creating a simulation box, specifying a) initial positions and velocities of all the atoms in the molecule in the lipid membrane system and lipid molecules are arranged in form of a membrane of type of lipid and the lipid molecule are inserted at a membrane midplane,

wherein the molecule in the solvent system and the molecule in the lipid membrane system are subjected to potential energy minimization, and overlaps between one or more pairs of atoms are removed;

generating (306) a trajectory comprising the position and velocities of all the atoms in the molecule in the solvent system and the molecule in the lipid membrane system, at all timesteps during the course of the plurality of molecular dynamic simulations; and

extracting (308) values of the first set of features and the second set of features, from the trajectory, wherein in the trajectory, each single configuration is referred to as a snapshot consisting of positions and velocities of all particles at a particular time instant, values of each of the features forming the first set of features and the second set of features are calculated as a function of the positions and velocities of the particles, and multiple snapshots are extracted from the generated trajectory and values are calculated thereof;

representing (210), via the one or more hardware processors, the pre-processed data set in a 2-Dimensional (2D) array format as time-series data with multiple features; and

generating (212), via the one or more hardware processors, a free energy of permeation by processing the pre-processed data set in the 2D array format using a machine learning data model, wherein the machine learning data model is generated by using data obtained for a plurality of molecule-lipid layer combinations that is different molecules and different lipid layers, wherein the data is further used to train the data model comprising long short term memory, LSTM, layers and an attention layer of a neural network, wherein each LSTM cell of the LSTM layer takes feature vector at each time step ($x_t$), hidden state of the previous cell ($h_{t-1}$), and previous cell state ($c_{t-1}$) as input, and produces cell state ($c_t$) and hidden state ($h_t$) as output, wherein the LSTM layer is a way of encoding the input so that subsequent layers learns information from the hidden states, comprising:

generating (212a) a context vector, by feeding hidden states from the LSTM layer into the attention layer, comprising information on a time dependency, a feature relevance inferring that various model weights used while building the data model are adjusted during a training process such that an output is sensitive to some features more than others, and a similarity, for the molecule and the lipid membrane in the pre-processed data set stored in the 2D array format, wherein the 2D array represents an evolution of the molecule in the solvent system and/or the molecule in the lipid membrane system with time and by moving across rows in the 2D array, information on how the features of the molecule in the solvent system and/or the molecule in the lipid membrane system changes with time is obtained referring to as the time dependency, wherein the context vector is calculated using the attention layer of the data model, and captures similarity between different molecules, wherein the context vector represents a relative importance of the features for computing the free energy of permeation and the context vector also reduces a sequential information into a simple vector that is mapped to output of the data model referring to the free energy of permeation, wherein the LSTM layer and the attention layer are sequentially used to learn the time dependency, the feature relevance, and the similarity between molecules, wherein during the training, deviations of free energy values calculated by the data model from actual values are backpropagated, and the model weights of all the layers are adjusted to minimize the deviation, wherein after training phase, the trained data model used for processing real-time inputs and for generating value of the free energy of permeation corresponding to values of the real-time inputs;

generating (212b) a transformed vector comprising information on non-linear dependency of the components of the context vector, and a dense layer learns the non-linear dependency of elements of the context vector with the free energy, wherein by passing the context vector through the dense layer, another vector of same length that incorporates the nonlinearity is obtained; and

estimating (212c) the free energy of permeation in an output neuron as weighted sum of components of the transformed vector of the dense layer,

wherein the data model is deployed to virtually screen small drug-like molecules, and the one or more hardware processors is designed to devise a solution to estimate the free energy of permeation of small molecules across membranes.

2. A system (100), comprising:

one or more hardware processors (102);
a communication interface (112); and
a memory (104) storing a plurality of instructions, wherein the plurality of instructions when executed, cause the one or more hardware processors to:

generate a first set of features based on interaction of a molecule with a lipid membrane, by performing a plurality of molecular dynamics simulations, wherein values of the first set of features are collected as a time series data, wherein the first set of features comprises a) interaction energy Lennard-Jones, LJ, of the molecule with the lipid membrane, b) area per lipid of the lipid membrane, c) root mean square (RMS) deviation of a plurality of lipid molecules from a corresponding initial position, and d) RMS deviation for combination of a plurality of lipid membrane and the molecule;
generate a second set of features based on interaction of the molecule with a solvent by performing the plurality of molecular dynamics simulations, wherein values of the second set of features are collected as a time series data, wherein the second set of features comprises a) surface area of the molecule that can be accessed by the solvent, b) molecule-solvent enthalpy, c) LJ interaction energy between the molecule and the solvent, and d) bond energy of the molecule;

generate a concatenated data set comprising values of the first set of features and the second set of features, extracted from a plurality of instances of the time series data of the first set of features and the second set of features, wherein generating the first set of features and the second set of features comprises:

generating (302) a molecule in a solvent system by specifying a) initial positions and velocities of all the atoms in the molecule in the solvent system, and b) a plurality of simulating conditions of the molecule in the solvent system, wherein simulations are performed at room temperature, 300K, or physiological temperature, 310K, atmospheric pressure, for pharmaceutical application, wherein the molecule in the solvent system is generated by creating a simulation box and inserting the molecule and solvent molecules in the simulation box, wherein inserting refers to specifying initial positions and velocities of all the atoms in the molecule in the solvent system and the molecule is inserted at the center of the simulation box and remaining box volume is filled up with solvent molecules;

generating (304) a molecule in a lipid membrane system by specifying a) initial positions and velocities of all the atoms in the molecule in the lipid membrane system, and b) a plurality of simulating conditions of the molecule in the lipid membrane system, wherein the molecule in the lipid membrane system is generated by creating a simulation box, specifying a) initial positions and velocities of all the atoms in the molecule in the lipid membrane system and lipid molecules are arranged in form of a membrane of type of lipid and the lipid molecule are inserted at a membrane midplane ,

wherein the molecule in the solvent system and the molecule in the lipid membrane system are subjected to potential energy minimization, and overlaps between one or more pairs of atoms are removed;

generating (306) a trajectory comprising the position and velocities of all the atoms in the molecule in the solvent system and the molecule in the lipid membrane system, at all timesteps during the course of the plurality of molecular dynamic simulations; and

extracting (308) values of the first set of features and the second set of features, from the trajectory, wherein in the trajectory, each single configuration is referred to as a snapshot consisting of positions and velocities of all particles at a particular time instant, values of each of the features forming the first set of features and the second set of features are calculated as a function of the positions and velocities of the particles, and multiple snapshots are extracted from the generated trajectory and values are calculated thereof;

pre-process the concatenated data set to generate a pre-processed data set;

represent the pre-processed data set in a 2-Dimensional (2D) array format as time-series data with multiple features; and generate a free energy of permeation by processing the pre-processed data set in the 2D array format using a machine learning data model, wherein the machine learning data model is generated by using data obtained for a plurality of molecule-lipid layer combinations that is different molecules and different lipid layers, wherein the data is further used to train the data model comprising long short term memory, LSTM, layers and an attention layer of a neural network, wherein each LSTM cell of the LSTM layer takes feature vector at each time step (xt), hidden state of the previous cell ($h_{t-1}$), and previous cell state ($c_{t-1}$) as input, and produces cell state (ct) and hidden state (ht) as output, wherein the LSTM layer is a way of encoding the input so that subsequent layers learns information from the hidden states, by:

generating a context vector, by feeding hidden states from the LSTM layer into the attention layer, comprising information on time dependency, feature relevance inferring that various model weights used while building the data model are adjusted during a training process such that an output is sensitive to some features more than others, and similarity, for the molecule and the lipid membrane in the pre-processed data set stored in the 2D array format, wherein the 2D array represents an evolution of the molecule in the solvent system and/or the molecule in the lipid membrane system with time and by moving across rows in the 2D array, information on how the features of the molecule in the solvent system and/or the molecule in the lipid membrane system changes with time is obtained referring to as the time dependency , wherein the context vector is calculated using the attention layer of the data model, and captures similarity between different molecules, wherein the context vector represents a relative importance of the features for computing the free energy of permeation and the context vector also reduces a sequential information into a simple vector that is mapped to output of the data model referring to the free energy of permeation, wherein the LSTM layer and the attention layer are sequentially used to learn the time dependency, the feature relevance, and the similarity between molecules, wherein during the training, deviations of free energy values calculated by the data model from actual values are backpropagated, and the model weights of all the layers are adjusted to minimize the deviation, wherein after training phase, the trained data model used for processing real-time inputs and for generating value of the free energy of permeation corresponding to values of the real-time inputs;

generating a transformed vector comprising information on non-linear dependency of the components of the context vector, and a dense layer learns the non-linear dependency of elements of the context vector with the free energy, wherein by passing the context vector through the dense layer, another vector of same length that incorporates the nonlinearity is obtained; and

estimating the free energy of permeation in an output neuron as weighted sum of components of the transformed vector of the dense layer,

wherein the data model is deployed to virtually screen small drug-like molecules, and the system (100) is designed to devise a solution to estimate the free energy of permeation of small molecules across membranes.

3. One or more non-transitory machine-readable information storage mediums comprising one or more instructions which when executed by one or more hardware processors cause:

generating a first set of features based on interaction of a molecule with a lipid membrane, by performing a plurality of molecular dynamics simulations, wherein values of the first set of features are collected as a time series data, wherein the first set of features comprises a) interaction energy Lennard-Jones, **LJ,** of the molecule with the lipid membrane, b) area per lipid of the lipid membrane, c) root mean square (RMS) deviation of a plurality of lipid molecules from a corresponding initial position, and d) RMS deviation for combination of a plurality of lipid membrane and the molecule;

generating a second set of features based on interaction of the molecule with a solvent by performing the plurality of molecular dynamics simulations, wherein values of the second set of features are collected as a time series data, wherein the second set of features comprises a) surface area of the molecule that can be accessed by the solvent, b) molecule-solvent enthalpy, c) LJ interaction energy between the molecule and the solvent, and d) bond energy of the molecule;

generating a concatenated data set comprising values of the first set of features and the second set of features, extracted from a plurality of instances of the time series data of the first set of features and the second set of features, wherein generating the first set of features and the second set of features comprises:

generating (302) a molecule in a solvent system by specifying a) initial positions and velocities of all the atoms in the molecule in the solvent system, and b) a plurality of simulating conditions of the molecule in the solvent system, wherein simulations are performed at room temperature, 300K, or physiological temperature, 310K, atmospheric pressure, for pharmaceutical application, wherein the molecule in the solvent system is generated by creating a simulation box and inserting the molecule and solvent molecules in the simulation box, wherein inserting refers to specifying initial positions and velocities of all the atoms in the molecule in the solvent system and the molecule is inserted at the center of the simulation box and remaining box volume is filled up with solvent molecules;

generating (304) a molecule in a lipid membrane system by specifying a) initial positions and velocities of all the atoms in the molecule in the lipid membrane system, and b) a plurality of simulating conditions of the molecule in the lipid membrane system, wherein the molecule in the lipid membrane system is generated by creating a simulation box, specifying a) initial positions and velocities of all the atoms in the molecule in the lipid membrane system and lipid molecules are arranged in form of a membrane of type of lipid and the lipid molecule are inserted at a membrane midplane ,

wherein the molecule in the solvent system and the molecule in the lipid membrane system are subjected to potential energy minimization, and overlaps between one or more pairs of atoms are removed;

generating (306) a trajectory comprising the position and velocities of all the atoms in the molecule in the solvent system and the molecule in the lipid membrane system, at all timesteps during the course of the plurality of molecular dynamic simulations; and

extracting (308) values of the first set of features and the second set of features, from the trajectory, wherein in the trajectory, each single configuration is referred to as a snapshot consisting of positions and velocities of all particles at a particular time instant, values of each of the features forming the first set of features and the second set of features are calculated as a function of the positions and velocities of the particles, and multiple snapshots are extracted from the generated trajectory and values are calculated thereof;

pre-processing the concatenated data set to generate a pre-processed data set;

representing the pre-processed data set in a 2-Dimensional array format as time-series data with multiple features; and

generating a free energy of permeation by processing the pre-processed data set in the 2D array format using a machine learning data model, wherein the machine learning data model is generated by using data obtained for a plurality of molecule-lipid layer combinations that is different molecules and different lipid layers, wherein the data is further used to train the data model comprising long short term memory, LSTM, layers and an attention layer of a neural network, wherein each LSTM cell of the LSTM layer takes feature vector at each time step (xt), hidden state of the previous cell ($h_{t-1}$), and previous cell state ($c_{t-1}$) as input, and produces cell state (ct) and hidden state (ht) as output, wherein the LSTM layer is a way of encoding the input so that subsequent layers learns information from the hidden states, comprising:

generating a context vector, by feeding hidden states from the LSTM layer into the attention layer, comprising information on a time dependency, a feature relevance inferring that various model weights used while building the data model are adjusted during a training process such that an output is sensitive to some features more than others, and a similarity, for the molecule and

the lipid membrane in the pre-processed data set stored in the 2D array format, wherein the 2D array represents an evolution of the molecule in the solvent system and/or the molecule in the lipid membrane system with time and by moving across rows in the 2D array, information on how the features of the molecule in the solvent system and/or the molecule in the lipid membrane system changes with time is obtained referring to as the time dependency , wherein the context vector is calculated using the attention layer of the data model, and captures similarity between different molecules, wherein the context vector represents a relative importance of the features for computing the free energy of permeation and the context vector also reduces a sequential information into a simple vector that is mapped to output of the data model referring to the free energy of permeation, wherein the LSTM layer and the attention layer are sequentially used to learn the time dependency, the feature relevance, and the similarity between molecules, wherein during the training, deviations of free energy values calculated by the data model from actual values are backpropagated, and the model weights of all the layers are adjusted to minimize the deviation, wherein after training phase, the trained data model used for processing real-time inputs and for generating value of the free energy of permeation corresponding to values of the real-time inputs;

generating a transformed vector comprising information on non-linear dependency of the components of the context vector, and a dense layer learns the non-linear dependency of elements of the context vector with the free energy, wherein by passing the context vector through the dense layer, another vector of same length that incorporates the nonlinearity is obtained; and

estimating the free energy of permeation in an output neuron as weighted sum of components of the transformed vector of the dense layer,

wherein the data model is deployed to virtually screen small drug-like molecules, and a system (100) is designed to devise a solution to estimate the free energy of permeation of small molecules across membranes.

**Patentansprüche**

1.  Prozessorimplementiertes Verfahren (200), umfassend:

Erzeugen (202), über einen oder mehrere Hardwareprozessoren, eines ersten Satzes von Merkmalen basierend auf einer Wechselwirkung eines Moleküls mit einer Lipidmembran durch Durchführen einer Mehrzahl von molekulardynamischen Simulationen, wobei Werte des ersten Satzes von Merkmalen als Zeitreihendaten gesammelt werden, wobei der erste Satz von Merkmalen umfasst: a) eine Lennard-Jones-Wechselwirkungsenergie, LJ, des Moleküls mit der Lipidmembran, b) eine Fläche pro Lipid der Lipidmembran, c) eine quadratische Mittelwertabweichung (RMS-Abweichung) einer Mehrzahl von Lipidmolekülen von einer entsprechenden Anfangsposition und d) eine RMS-Abweichung für eine Kombination einer Mehrzahl von Lipidmembranen und des Moleküls;
Erzeugen (204), über den einen oder die mehreren Hardwareprozessoren, eines zweiten Satzes von Merkmalen basierend auf einer Wechselwirkung des Moleküls mit einem Lösungsmittel durch Durchführen der Mehrzahl von molekulardynamischen Simulationen, wobei Werte des zweiten Satzes von Merkmalen als Zeitreihendaten gesammelt werden, wobei der zweite Satz von Merkmalen umfasst: a) eine Oberfläche des Moleküls, auf die durch das Lösungsmittel zugegriffen werden kann, b) eine Molekül-Lösungsmittel-Enthalpie, c) eine LJ-Wechselwirkungsenergie zwischen dem Molekül und dem Lösungsmittel und d) eine Bindungsenergie des Moleküls;
Erzeugen (206), über den einen oder die mehreren Hardwareprozessoren, eines verketteten Datensatzes, der Werte des ersten Satzes von Merkmalen und des zweiten Satzes von Merkmalen umfasst, die aus einer Mehrzahl von Instanzen der Zeitreihendaten des ersten Satzes von Merkmalen und des zweiten Satzes von Merkmalen extrahiert werden;

Vorverarbeiten (208), über den einen oder die mehreren Hardwareprozessoren, des verketteten Datensatzes, um einen vorverarbeiteten Datensatz zu erzeugen, wobei das Erzeugen des ersten Satzes von Merkmalen und des zweiten Satzes von Merkmalen umfasst:

Erzeugen (302) eines Moleküls in einem Lösungsmittelsystem durch Spezifizieren von a) Anfangspositionen und -geschwindigkeiten aller Atome in dem Molekül in dem Lösungsmittelsystem und b) einer Mehrzahl von Simulationsbedingungen des Moleküls in dem Lösungsmittelsystem, wobei Simulationen bei Raumtemperatur, 300 K, oder physiologischer Temperatur, 310 K, Atmosphärendruck, für eine pharmazeutische Anwendung durchgeführt werden, wobei das Molekül in dem Lösungsmittelsystem durch Erzeugen einer Simulationsbox und Einfügen des Moleküls und der Lösungsmittelmoleküle in die Simulationsbox erzeugt wird, wobei sich das Einfügen auf das Spezifizieren von Anfangspositionen und -geschwindigkeiten aller Atome in dem Molekül in dem Lösungsmittelsystem bezieht und das Molekül in der Mitte der Simulationsbox eingefügt wird und das verbleibende Boxvolumen mit Lösungsmittelmolekülen gefüllt wird;

Erzeugen (304) eines Moleküls in einem Lipidmembransystem durch Spezifizieren von a) Anfangspositionen und -geschwindigkeiten aller Atome in dem Molekül in dem Lipidmembransystem und b) einer Mehrzahl von Simulationsbedingungen des Moleküls in dem Lipidmembransystem, wobei das Molekül in dem Lipidmembransystem durch Erzeugen einer Simulationsbox erzeugt wird, die a) Anfangspositionen und -geschwindigkeiten aller Atome in dem Molekül in dem Lipidmembransystem spezifiziert und Lipidmoleküle in Form einer Membran vom Lipidtyp angeordnet sind und das Lipidmolekül an einer Membranmittelebene eingefügt wird,

wobei das Molekül in dem Lösungsmittelsystem und das Molekül in dem Lipidmembransystem einer potentiellen Energieminimierung unterzogen werden und Überlappungen zwischen einem oder mehreren Paaren von Atomen entfernt werden;

Erzeugen (306) einer Trajektorie, die die Position und die Geschwindigkeiten aller Atome in dem Molekül in dem Lösungsmittelsystem und das Molekül in dem Lipidmembransystem umfasst, zu allen Zeitschritten während des Verlaufs der Mehrzahl von molekulardynamischen Simulationen; und

Extrahieren (308) von Werten des ersten Satzes von Merkmalen und des zweiten Satzes von Merkmalen aus der Trajektorie, wobei in der Trajektorie jede einzelne Konfiguration als eine Momentaufnahme bezeichnet wird, die aus Positionen und Geschwindigkeiten aller Partikel zu einem bestimmten Zeitpunkt besteht, Werte jedes der Merkmale, die den ersten Satz von Merkmalen und den zweiten Satz von Merkmalen bilden, als eine Funktion der Positionen und Geschwindigkeiten der Partikel berechnet werden und mehrere Momentaufnahmen aus der erzeugten Trajektorie extrahiert werden und Werte davon berechnet werden;

Darstellen (210), über den einen oder die mehreren Hardwareprozessoren, des vorverarbeiteten Datensatzes in einem 2-dimensionalen (2D) Array-Format als Zeitreihendaten mit mehreren Merkmalen; und

Erzeugen (212), über den einen oder die mehreren Hardwareprozessoren, einer freien Energie der Permeation durch Verarbeiten des vorverarbeiteten Datensatzes in dem 2D-Array-Format unter Verwendung eines maschinellen Lerndatenmodells, wobei das maschinelle Lerndatenmodell unter Verwendung von Daten erzeugt wird, die für eine Mehrzahl von Molekül-Lipidschicht-Kombinationen erhalten werden, die unterschiedliche Moleküle und unterschiedliche Lipidschichten sind, wobei die Daten ferner verwendet werden, um das Datenmodell zu trainieren, das Long-Short-Term-Memory-, LSTM-, Schichten und eine Aufmerksamkeitsschicht eines neuronalen Netzwerks umfasst, wobei jede LSTM-Zelle der LSTM-Schicht einen Merkmalsvektor zu jedem Zeitschritt (xt), einen verborgenen Zustand der vorherigen Zelle ($h_{t-1}$) und einen vorherigen Zellzustand ($c_{t-1}$) als Eingabe nimmt und einen Zellzustand (ct) und einen verborgenen Zustand (ht) als Ausgabe erzeugt, wobei die LSTM-Schicht eine Möglichkeit zum Codieren der Eingabe ist, so dass nachfolgende Schichten Informationen aus den verborgenen Zuständen lernen, umfassend:

Erzeugen (212a) eines Kontextvektors durch Einspeisen verborgener Zustände von der LSTM-Schicht in die Aufmerksamkeitsschicht, umfassend Informationen über eine Zeitabhängigkeit, eine Merkmalsrelevanz, die ableitet, dass verschiedene Modellgewichte, die während des Aufbauens des Datenmodells verwendet werden, während eines Trainingsprozesses so angepasst werden, dass eine Ausgabe für einige Merkmale empfindlicher ist als andere, und eine Ähnlichkeit für das Molekül und die Lipidmembran in dem vorverarbeiteten Datensatz, der in dem 2D-Array-Format gespeichert ist, wobei das 2D-Array eine Entwicklung des Moleküls in dem Lösungsmittelsystem und/oder des Moleküls in dem Lipidmembransystem mit der Zeit darstellt und durch Bewegen über Reihen in dem 2D-Array Informationen darüber, wie sich die Merkmale des Moleküls in dem Lösungsmittelsystem und/oder des Moleküls in dem Lipidmembransystem mit der Zeit ändern, unter Bezugnahme auf die Zeitabhängigkeit erhalten werden, wobei der Kontextvektor unter Verwendung der Aufmerksamkeitsschicht des Datenmodells berechnet wird und eine Ähnlichkeit zwischen unterschiedlichen Molekülen erfasst, wobei der Kontextvektor eine relative Wichtigkeit der Merkmale zum Berechnen der freien Energie der Permeation darstellt und der Kontextvektor auch eine sequentielle Information in einen einfachen Vektor reduziert, der auf eine Ausgabe des Datenmodells unter Bezugnahme auf die freie Energie der Permeation abgebildet wird, wobei die LSTM-Schicht und die Aufmerksamkeitsschicht sequentiell verwendet werden, um die Zeitabhängigkeit, die Merkmalsrelevanz und die Ähnlichkeit zwischen Molekülen zu lernen, wobei während des Trainings Abweichungen von Werten der freien Energie, die durch das Datenmodell berechnet werden, von tatsächlichen Werten rückpropagiert werden und die Modellgewichte aller Schichten angepasst werden, um die Abweichung zu minimieren, wobei nach der Trainingsphase das trainierte Datenmodell zum Verarbeiten von Echtzeiteingaben und zum Erzeugen eines Werts der freien Energie der Permeation entsprechend Werten der Echtzeiteingaben verwendet wird;

Erzeugen (212b) eines transformierten Vektors, der Informationen über nichtlineare Abhängigkeit der Komponenten des Kontextvektors umfasst, und einer dichten Schicht, die die nichtlineare Abhängigkeit von Elementen des Kontextvektors mit der freien Energie erlernt, wobei durch Leiten des Kontextvektors durch die dichte Schicht ein anderer Vektor derselben Länge, der die Nichtlinearität enthält, erhalten wird; und

Schätzen (212c) der freien Energie der Permeation in einem Ausgabeneuron als gewichtete Summe von Komponenten des transformierten Vektors der dichten Schicht,

wobei das Datenmodell eingesetzt wird, um kleine arzneimittelartige Moleküle virtuell zu screenen, und der eine oder die mehreren Hardwareprozessoren entworfen sind, um eine Lösung zu entwickeln, um die freie Energie der Permeation kleiner Moleküle über Membranen zu schätzen.

2. System (100), umfassend:

einen oder mehrere Hardwareprozessoren (102);
eine Kommunikationsschnittstelle (112); und
einen Speicher (104), der eine Mehrzahl von Anweisungen speichert, wobei die Mehrzahl von Anweisungen, wenn sie ausgeführt werden, den einen oder die mehreren Hardwareprozessoren veranlassen zum:

Erzeugen eines ersten Satzes von Merkmalen basierend auf einer Wechselwirkung eines Moleküls mit einer Lipidmembran durch Durchführen einer Mehrzahl von moleculardynamischen Simulationen, wobei Werte des ersten Satzes von Merkmalen als Zeitreihendaten gesammelt werden, wobei der erste Satz von Merkmalen umfasst: a) eine Lennard-Jones-Wechselwirkungsenergie, LJ, des Moleküls mit der Lipidmembran, b) eine Fläche pro Lipid der Lipidmembran, c) eine quadratische Mittelwertabweichung (RMS-Abweichung) einer Mehrzahl von Lipidmolekülen von einer entsprechenden Anfangsposition und d) eine RMS-Abweichung für eine Kombination einer Mehrzahl von Lipidmembranen und des Moleküls;

Erzeugen eines zweiten Satzes von Merkmalen basierend auf einer Wechselwirkung des Moleküls mit einem Lösungsmittel durch Durchführen der Mehrzahl von moleculardynamischen Simulationen, wobei Werte des zweiten Satzes von Merkmalen als Zeitreihendaten gesammelt werden, wobei der zweite Satz von Merkmalen umfasst: a) eine Oberfläche des Moleküls, auf die durch das Lösungsmittel zugegriffen werden kann, b) eine Molekül-Lösungsmittel-Enthalpie, c) eine **LJ**-Wechselwirkungsenergie zwischen dem Molekül und dem Lösungsmittel und d) eine Bindungsenergie des Moleküls;

Erzeugen eines verketteten Datensatzes, der Werte des ersten Satzes von Merkmalen und des zweiten Satzes von Merkmalen umfasst, die aus einer Mehrzahl von Instanzen der Zeitreihendaten des ersten Satzes von Merkmalen und des zweiten Satzes von Merkmalen extrahiert werden, wobei das Erzeugen des ersten Satzes von Merkmalen und des zweiten Satzes von Merkmalen umfasst:

Erzeugen (302) eines Moleküls in einem Lösungsmittelsystem durch Spezifizieren von a) Anfangspositionen und -geschwindigkeiten aller Atome in dem Molekül in dem Lösungsmittelsystem und b) einer Mehrzahl von Simulationsbedingungen des Moleküls in dem Lösungsmittelsystem, wobei Simulationen bei Raumtemperatur, 300 K, oder physiologischer Temperatur, 310 K, Atmosphärendruck, für eine pharmazeutische Anwendung durchgeführt werden, wobei das Molekül in dem Lösungsmittelsystem durch Erzeugen einer Simulationsbox und Einfügen des Moleküls und der Lösungsmittelmoleküle in die Simulationsbox erzeugt wird, wobei sich das Einfügen auf das Spezifizieren von Anfangspositionen und -geschwindigkeiten aller Atome in dem Molekül in dem Lösungsmittelsystem bezieht und das Molekül in der Mitte der Simulationsbox eingefügt wird und das verbleibende Boxvolumen mit Lösungsmittelmolekülen gefüllt wird;

Erzeugen (304) eines Moleküls in einem Lipidmembransystem durch Spezifizieren von a) Anfangspositionen und -geschwindigkeiten aller Atome in dem Molekül in dem Lipidmembransystem und b) einer Mehrzahl von Simulationsbedingungen des Moleküls in dem Lipidmembransystem, wobei das Molekül in dem Lipidmembransystem durch Erzeugen einer Simulationsbox erzeugt wird, die a) Anfangspositionen und -geschwindigkeiten aller Atome in dem Molekül in dem Lipidmembransystem spezifiziert und Lipidmoleküle in Form einer Membran vom Lipidtyp angeordnet sind und das Lipidmolekül an einer Membranmittelebene eingefügt wird,

wobei das Molekül in dem Lösungsmittelsystem und das Molekül in dem Lipidmembransystem einer potentiellen Energieminimierung unterzogen werden und Überlappungen zwischen einem oder mehreren Paaren von Atomen entfernt werden;

Erzeugen (306) einer Trajektorie, die die Position und die Geschwindigkeiten aller Atome in dem Molekül in dem Lösungsmittelsystem und das Molekül in dem Lipidmembransystem umfasst, zu allen Zeitschritten während des Verlaufs der Mehrzahl von molekulardynamischen Simulationen; und

Extrahieren (308) von Werten des ersten Satzes von Merkmalen und des zweiten Satzes von Merkmalen aus der Trajektorie, wobei in der Trajektorie jede einzelne Konfiguration als eine Momentaufnahme bezeichnet wird, die aus Positionen und Geschwindigkeiten aller Partikel zu einem bestimmten Zeitpunkt besteht, Werte jedes der Merkmale, die den ersten Satz von Merkmalen und den zweiten Satz von Merkmalen bilden, als eine Funktion der Positionen und Geschwindigkeiten der Partikel berechnet werden und mehrere Momentaufnahmen aus der erzeugten Trajektorie extrahiert werden und Werte davon berechnet werden;

Vorverarbeiten des verketteten Datensatzes, um einen vorverarbeiteten Datensatz zu erzeugen;

Darstellen des vorverarbeiteten Datensatzes in einem 2-dimensionalen (2D) Array-Format als Zeitreihendaten mit mehreren Merkmalen; und

Erzeugen einer freien Energie der Permeation durch Verarbeiten des vorverarbeiteten Datensatzes in dem 2D-Array-Format unter Verwendung eines maschinellen Lerndatenmodells, wobei das maschinelle Lerndatenmodell unter Verwendung von Daten erzeugt wird, die für eine Mehrzahl von Molekül-Lipidschicht-Kombinationen erhalten werden, die unterschiedliche Moleküle und unterschiedliche Lipidschichten sind, wobei die Daten ferner verwendet werden, um das Datenmodell zu trainieren, das Long-Short-Term-Memory-, LSTM-, Schichten und eine Aufmerksamkeitsschicht eines neuronalen Netzwerks umfasst, wobei jede LSTM-Zelle der LSTM-Schicht einen Merkmalsvektor zu jedem Zeitschritt (xt), einen verborgenen Zustand der vorherigen Zelle ($h_{t-1}$) und einen vorherigen Zellzustand ($c_{t-1}$) als Eingabe nimmt und einen Zellzustand (ct) und einen verborgenen Zustand (ht) als Ausgabe erzeugt, wobei die LSTM-Schicht eine Möglichkeit zum Codieren der Eingabe ist, so dass nachfolgende Schichten Informationen aus den verborgenen Zuständen lernen, durch:

Erzeugen eines Kontextvektors durch Einspeisen verborgener Zustände von der LSTM-Schicht in die Aufmerksamkeitsschicht, der Informationen über eine Zeitabhängigkeit, eine Merkmalsrelevanz, die ableitet, dass verschiedene Modellgewichte, die verwendet werden, während das Datenmodell aufgebaut wird, während eines Trainingsprozesses angepasst werden, so dass eine Ausgabe für einige Merkmale empfindlicher als andere ist, und eine Ähnlichkeit für das Molekül und die Lipidmembran in dem vorverarbeiteten Datensatz, der in dem 2D-Array-Format gespeichert ist, umfasst, wobei das 2D-Array eine Entwicklung des Moleküls in dem Lösungsmittelsystem und/oder des Moleküls in dem Lipidmembransystem mit der Zeit darstellt und durch Bewegen über Reihen in dem 2D-Array Informationen darüber, wie sich die Merkmale des Moleküls in dem Lösungsmittelsystem und/oder des Moleküls in dem Lipidmembransystem mit der Zeit ändern, unter Bezugnahme auf die Zeitabhängigkeit erhalten werden, wobei der Kontextvektor unter Verwendung der Aufmerksamkeitsschicht des Datenmodells berechnet wird und eine Ähnlichkeit zwischen unterschiedlichen Molekülen erfasst, wobei der Kontextvektor eine relative Wichtigkeit der Merkmale zum Berechnen der freien Energie der Permeation darstellt und der Kontextvektor auch sequentielle Informationen in einen einfachen Vektor reduziert, der auf eine Ausgabe des Datenmodells unter Bezugnahme auf die freie Energie der Permeation abgebildet wird, wobei die LSTM-Schicht und die Aufmerksamkeitsschicht sequentiell verwendet werden, um die Zeitabhängigkeit, die Merkmalsrelevanz und die Ähnlichkeit zwischen Molekülen zu lernen, wobei während des Trainings Abweichungen von Werten der freien Energie, die durch das Datenmodell berechnet werden, von tatsächlichen Werten rückpropagiert werden und die Modellgewichte aller Schichten angepasst werden, um die Abweichung zu minimieren, wobei nach der Trainingsphase das trainierte Datenmodell zum Verarbeiten von Echtzeiteingaben und zum Erzeugen eines Werts der freien Energie der Permeation, der Werten der Echtzeiteingaben entspricht, verwendet wird;

Erzeugen eines transformierten Vektors, der Informationen über nichtlineare Abhängigkeit der Komponenten des Kontextvektors umfasst, und einer dichten Schicht, die die nichtlineare Abhängigkeit von Elementen des Kontextvektors mit der freien Energie erlernt, wobei durch Leiten des Kontextvektors durch die dichte Schicht ein anderer Vektor derselben Länge, der die Nichtlinearität enthält, erhalten wird; und

Schätzen der freien Energie der Permeation in einem Ausgabeneuron als gewichtete Summe von Komponenten des transformierten Vektors der dichten Schicht,

wobei das Datenmodell eingesetzt wird, um kleine arzneimittelartige Moleküle virtuell zu screenen, und das System (100) entworfen ist, um eine Lösung zu entwickeln, um die freie Energie der Permeation kleiner Moleküle über Membranen zu schätzen.

3. Ein oder mehrere nichtflüchtige maschinenlesbare Informationsspeichermedien, die eine oder mehrere Anweisungen umfassen, die, wenn sie von einem oder mehreren Hardwareprozessoren ausgeführt werden, veranlassen zum:

Erzeugen eines ersten Satzes von Merkmalen basierend auf einer Wechselwirkung eines Moleküls mit einer Lipidmembran durch Durchführen einer Mehrzahl von moleculardynamischen Simulationen, wobei Werte des ersten Satzes von Merkmalen als Zeitreihendaten gesammelt werden, wobei der erste Satz von Merkmalen umfasst: a) eine Lennard-Jones-Wechselwirkungsenergie, LJ, des Moleküls mit der Lipidmembran, b) eine Fläche pro Lipid der Lipidmembran, c) eine quadratische Mittelwertabweichung (RMS-Abweichung) einer Mehrzahl von Lipidmolekülen von einer entsprechenden Anfangsposition und d) eine RMS-Abweichung für eine Kombination einer Mehrzahl von Lipidmembranen und des Moleküls;

Erzeugen eines zweiten Satzes von Merkmalen basierend auf einer Wechselwirkung des Moleküls mit einem Lösungsmittel durch Durchführen der Mehrzahl von moleculardynamischen Simulationen, wobei Werte des zweiten Satzes von Merkmalen als Zeitreihendaten gesammelt werden, wobei der zweite Satz von Merkmalen umfasst: a) eine Oberfläche des Moleküls, auf die durch das Lösungsmittel zugegriffen werden kann, b) eine Molekül-Lösungsmittel-Enthalpie, c) eine LJ-Wechselwirkungsenergie zwischen dem Molekül und dem Lösungsmittel und d) eine Bindungsenergie des Moleküls;

Erzeugen eines verketteten Datensatzes, der Werte des ersten Satzes von Merkmalen und des zweiten Satzes von Merkmalen umfasst, die aus einer Mehrzahl von Instanzen der Zeitreihendaten des ersten Satzes von Merkmalen und des zweiten Satzes von Merkmalen extrahiert werden, wobei das Erzeugen des ersten Satzes von Merkmalen und des zweiten Satzes von Merkmalen umfasst:

Erzeugen (302) eines Moleküls in einem Lösungsmittelsystem durch Spezifizieren von a) Anfangspositionen und -geschwindigkeiten aller Atome in dem Molekül in dem Lösungsmittelsystem und b) einer Mehrzahl von Simulationsbedingungen des Moleküls in dem Lösungsmittelsystem, wobei Simulationen bei Raumtemperatur, 300 K, oder physiologischer Temperatur, 310 K, Atmosphärendruck, für eine pharmazeutische Anwendung durchgeführt werden, wobei das Molekül in dem Lösungsmittelsystem durch Erzeugen einer Simulationsbox und Einfügen des Moleküls und der Lösungsmittelmoleküle in die Simulationsbox erzeugt wird, wobei sich das Einfügen auf das Spezifizieren von Anfangspositionen und -geschwindigkeiten aller Atome in dem Molekül in dem Lösungsmittelsystem bezieht und das Molekül in der Mitte der Simulationsbox eingefügt wird und das verbleibende Boxvolumen mit Lösungsmittelmolekülen gefüllt wird;

Erzeugen (304) eines Moleküls in einem Lipidmembransystem durch Spezifizieren von a) Anfangspositionen und -geschwindigkeiten aller Atome in dem Molekül in dem Lipidmembransystem und b) einer Mehrzahl von Simulationsbedingungen des Moleküls in dem Lipidmembransystem, wobei das Molekül in dem Lipidmembransystem durch Erzeugen einer Simulationsbox erzeugt wird, die a) Anfangspositionen und -geschwindigkeiten aller Atome in dem Molekül in dem Lipidmembransystem spezifiziert und Lipidmoleküle in Form einer Membran vom Lipidtyp angeordnet sind und das Lipidmolekül an einer Membranmittelebene eingefügt wird,

wobei das Molekül in dem Lösungsmittelsystem und das Molekül in dem Lipidmembransystem einer potentiellen Energieminimierung unterzogen werden und Überlappungen zwischen einem oder mehreren Paaren von Atomen entfernt werden;

Erzeugen (306) einer Trajektorie, die die Position und die Geschwindigkeiten aller Atome in dem Molekül in dem Lösungsmittelsystem und das Molekül in dem Lipidmembransystem umfasst, zu allen Zeitschritten während des Verlaufs der Mehrzahl von molekulardynamischen Simulationen; und

Extrahieren (308) von Werten des ersten Satzes von Merkmalen und des zweiten Satzes von Merkmalen aus der Trajektorie, wobei in der Trajektorie jede einzelne Konfiguration als eine Momentaufnahme bezeichnet wird, die aus Positionen und Geschwindigkeiten aller Partikel zu einem bestimmten Zeitpunkt besteht, Werte jedes der Merkmale, die den ersten Satz von Merkmalen und den zweiten Satz von Merkmalen bilden, als eine Funktion der Positionen und Geschwindigkeiten der Partikel berechnet werden und mehrere Momentaufnahmen aus der erzeugten Trajektorie extrahiert werden und Werte davon berechnet werden;

Vorverarbeiten des verketteten Datensatzes, um einen vorverarbeiteten Datensatz zu erzeugen;
Darstellen des vorverarbeiteten Datensatzes in einem 2-dimensionalen Array-Format als Zeitreihendaten mit mehreren Merkmalen; und
Erzeugen einer freien Energie der Permeation durch Verarbeiten des vorverarbeiteten Datensatzes in dem 2D-Array-Format unter Verwendung eines maschinellen Lerndatenmodells, wobei das maschinelle Lerndatenmodell unter Verwendung von Daten erzeugt wird, die für eine Mehrzahl von Molekül-Lipidschicht-Kombinationen erhalten werden, die unterschiedliche Moleküle und unterschiedliche Lipidschichten sind, wobei die Daten ferner verwendet werden, um das Datenmodell zu trainieren, das Long-Short-Term-Memory-, LSTM-, Schichten und eine Aufmerksamkeitsschicht eines neuronalen Netzwerks umfasst, wobei jede LSTM-Zelle der LSTM-Schicht einen Merkmalsvektor zu jedem Zeitschritt (xt), einen verborgenen Zustand der vorherigen Zelle ($h_{t-1}$) und einen vorherigen Zellzustand ($c_{t-1}$) als Eingabe nimmt und einen Zellzustand ($c_t$) und einen verborgenen Zustand ($h_t$) als Ausgabe erzeugt, wobei die LSTM-Schicht eine Möglichkeit zum Codieren der Eingabe ist, so dass nachfolgende Schichten Informationen aus den verborgenen Zuständen lernen, umfassend:

Erzeugen eines Kontextvektors durch Einspeisen verborgener Zustände von der LSTM-Schicht in die Aufmerksamkeitsschicht, umfassend Informationen über eine Zeitabhängigkeit, eine Merkmalsrelevanz, die ableitet, dass verschiedene Modellgewichte, die während des Aufbauens des Datenmodells verwendet werden, während eines Trainingsprozesses so angepasst werden, dass eine Ausgabe für einige Merkmale empfindlicher ist als andere, und eine Ähnlichkeit für das Molekül und die Lipidmembran in dem vorverarbeiteten Datensatz, der in dem 2D-Array-Format gespeichert ist, wobei das 2D-Array eine Entwicklung des Moleküls in dem Lösungsmittelsystem und/oder des Moleküls in dem Lipidmembransystem mit der Zeit darstellt und durch Bewegen über Reihen in dem 2D-Array Informationen darüber, wie sich die Merkmale des Moleküls in dem Lösungsmittelsystem und/oder des Moleküls in dem Lipidmembransystem mit der Zeit ändern, unter Bezugnahme auf die Zeitabhängigkeit erhalten werden, wobei der Kontextvektor unter Verwendung der Aufmerksamkeitsschicht des Datenmodells berechnet wird und die Ähnlichkeit zwischen unterschiedlichen Molekülen erfasst, wobei der Kontextvektor eine relative Wichtigkeit der Merkmale zum Berechnen der freien Energie der Permeation darstellt und der Kontextvektor auch eine sequentielle Information in einen einfachen Vektor reduziert, der auf die Ausgabe des Datenmodells unter Bezugnahme auf die freie Energie der Permeation abgebildet wird, wobei die LSTM-Schicht und die Aufmerksamkeitsschicht sequentiell verwendet werden, um die Zeitabhängigkeit, die Merkmalsrelevanz und die Ähnlichkeit zwischen Molekülen zu lernen, wobei während des Trainings Abweichungen von Werten der freien Energie, die durch das Datenmodell berechnet werden, von tatsächlichen Werten rückpropagiert werden und die Modellgewichte aller Schichten angepasst werden, um die Abweichung zu minimieren, wobei nach der Trainingsphase das trainierte Datenmodell zum Verarbeiten von Echtzeiteingaben und zum Erzeugen eines Werts der freien Energie der Permeation entsprechend den Werten der Echtzeiteingaben verwendet wird;
Erzeugen eines transformierten Vektors, der Informationen über nichtlineare Abhängigkeit der Komponenten des Kontextvektors umfasst, und einer dichten Schicht, die die nichtlineare Abhängigkeit von Elementen des Kontextvektors mit der freien Energie erlernt, wobei durch Leiten des Kontextvektors durch die dichte Schicht ein anderer Vektor derselben Länge, der die Nichtlinearität enthält, erhalten wird; und
Schätzen der freien Energie der Permeation in einem Ausgabeneuron als gewichtete Summe von Komponenten des transformierten Vektors der dichten Schicht,
wobei das Datenmodell eingesetzt wird, um kleine arzneimittelartige Moleküle virtuell zu screenen, und ein System (100) entworfen ist, um eine Lösung zu entwickeln, um die freie Energie der Permeation kleiner Moleküle über Membranen zu schätzen.

## Revendications

1. Procédé mis en œuvre par processeur (200), comprenant :

la génération (202), par l'intermédiaire d'un ou plusieurs processeurs matériels, d'un premier ensemble de caractéristiques sur la base d'une interaction d'une molécule avec une membrane lipidique, en effectuant une pluralité de simulations de dynamique moléculaire, dans lequel des valeurs du premier ensemble de caractéristiques sont collectées en tant que données de série temporelle, dans lequel le premier ensemble de caractéristiques comprend a) une énergie d'interaction de Lennard-Jones, LJ, de la molécule avec la membrane lipidique, b) une aire par lipide de la membrane lipidique, c) un écart quadratique moyen (RMS) d'une pluralité de molécules lipidiques par rapport à une position initiale correspondante, et d) un écart RMS pour une combinaison d'une pluralité de membranes lipidiques et de la molécule ;

la génération (204), par l'intermédiaire des un ou plusieurs processeurs matériels, d'un second ensemble de caractéristiques sur la base d'une interaction de la molécule avec un solvant en effectuant la pluralité de simulations de dynamique moléculaire, dans lequel des valeurs du second ensemble de caractéristiques sont collectées en tant que données de série temporelle, dans lequel le second ensemble de caractéristiques comprend a) une aire de surface de la molécule à laquelle le solvant peut accéder, b) une enthalpie molécule-solvant, c) une énergie d'interaction LJ entre la molécule et le solvant, et d) une énergie de liaison de la molécule ;

la génération (206), par l'intermédiaire des un ou plusieurs processeurs matériels, d'un ensemble de données concaténé comprenant des valeurs du premier ensemble de caractéristiques et du second ensemble de caractéristiques, extraites d'une pluralité d'instances des données de série temporelle du premier ensemble de caractéristiques et du second ensemble de caractéristiques ;

le prétraitement (208), par l'intermédiaire des un ou plusieurs processeurs matériels, de l'ensemble de données concaténé pour générer un ensemble de données prétraité, dans lequel la génération du premier ensemble de caractéristiques et du second ensemble de caractéristiques comprend :

la génération (302) d'une molécule dans un système de solvant en spécifiant a) des positions et des vitesses initiales de tous les atomes dans la molécule dans le système de solvant, et b) une pluralité de conditions de simulation de la molécule dans le système de solvant, dans lequel des simulations sont effectuées à température ambiante, 300 K, ou température physiologique, 310 K, pression atmosphérique, pour une application pharmaceutique, dans lequel la molécule dans le système de solvant est générée en créant une boîte de simulation et en insérant la molécule et les molécules de solvant dans la boîte de simulation, dans lequel l'insertion se réfère à la spécification de positions et de vitesses initiales de tous les atomes dans la molécule dans le système de solvant et la molécule est insérée au centre de la boîte de simulation et le volume de boîte restant est rempli de molécules de solvant ;

la génération (304) d'une molécule dans un système de membrane lipidique en spécifiant a) des positions et des vitesses initiales de tous les atomes dans la molécule dans le système de membrane lipidique, et b) une pluralité de conditions de simulation de la molécule dans le système de membrane lipidique, dans lequel la molécule dans le système de membrane lipidique est générée en créant une boîte de simulation, en spécifiant a) des positions et des vitesses initiales de tous les atomes dans la molécule dans le système de membrane lipidique et des molécules lipidiques sont agencées sous la forme d'une membrane de type lipide et la molécule lipidique est insérée au niveau d'un plan médian de membrane,

dans lequel la molécule dans le système de solvant et la molécule dans le système de membrane lipidique sont soumises à une minimisation d'énergie potentielle, et des chevauchements entre une ou plusieurs paires d'atomes sont éliminés ;

la génération (306) d'une trajectoire comprenant la position et les vitesses de tous les atomes dans la molécule dans le système de solvant et la molécule dans le système de membrane lipidique, à tous les pas de temps au cours de la pluralité de simulations dynamiques moléculaires ; et

l'extraction (308) de valeurs du premier ensemble de caractéristiques et du second ensemble de caractéristiques, à partir de la trajectoire, dans lequel, dans la trajectoire, chaque configuration unique est appelée un instantané constitué de positions et de vitesses de toutes les particules à un instant particulier, des valeurs de chacune des caractéristiques formant le premier ensemble de caractéristiques et le second ensemble de caractéristiques sont calculées en fonction des positions et des vitesses des particules, et de multiples instantanés sont extraits de la trajectoire générée et des valeurs de ceux-ci sont calculées ;

la représentation (210), par l'intermédiaire des un ou plusieurs processeurs matériels, de l'ensemble de données prétraité dans un format de réseau bidimensionnel (2D) en tant que données de série temporelle avec de multiples caractéristiques ; et

la génération (212), par l'intermédiaire des un ou plusieurs processeurs matériels, d'une énergie libre de perméation en traitant l'ensemble de données prétraité dans le format de réseau 2D à l'aide d'un modèle de données d'apprentissage automatique, dans lequel le modèle de données d'apprentissage automatique est généré à l'aide de données obtenues pour une pluralité de combinaisons molécule-couche lipidique qui sont des molécules différentes et des couches lipidiques différentes, dans lequel les données sont en outre utilisées pour entraîner le modèle de données comprenant des couches de mémoire à long terme, LSTM, et une couche d'attention d'un réseau neuronal, dans lequel chaque cellule LSTM de la couche LSTM prend un vecteur de caractéristique à chaque pas de temps (xt), un état caché de la cellule précédente ($h_{t-1}$), et un état de cellule précédent ($c_{t-1}$) en tant qu'entrée, et produit un état de cellule ($c_t$) et un état caché ($h_t$) en tant que sortie, dans lequel la couche LSTM est un moyen de codage de l'entrée de sorte que des couches suivantes apprennent des informations à partir des états cachés, comprenant :

la génération (212a) d'un vecteur de contexte, en alimentant des états cachés de la couche LSTM dans la couche d'attention, comprenant des informations sur une dépendance temporelle, une pertinence de caractéristique inférant que divers poids de modèle utilisés lors de la construction du modèle de données sont ajustés pendant un processus d'entraînement de sorte qu'une sortie est sensible à certaines caractéristiques plus que d'autres, et une similarité, pour la molécule et la membrane lipidique dans l'ensemble de données prétraité stocké dans le format de réseau 2D, dans lequel le réseau 2D représente une évolution de la molécule dans le système de solvant et/ou de la molécule dans le système de membrane lipidique avec le temps et en se déplaçant à travers des rangées dans le réseau 2D, des informations sur la façon dont les caractéristiques de la molécule dans le système de solvant et/ou de la molécule dans le système de membrane lipidique changent avec le temps sont obtenues en faisant référence à la dépendance temporelle, dans lequel le vecteur de contexte est calculé à l'aide de la couche d'attention du modèle de données, et capture une similarité entre différentes molécules, dans lequel le vecteur de contexte représente une importance relative des caractéristiques pour calculer l'énergie libre de perméation et le vecteur de contexte réduit également des informations séquentielles en un vecteur simple qui est mappé à la sortie du modèle de données faisant référence à l'énergie libre de perméation, dans lequel la couche LSTM et la couche d'attention sont utilisées séquentiellement pour apprendre la dépendance temporelle, la pertinence de caractéristique, et la similarité entre des molécules, dans lequel pendant l'entraînement, des écarts de valeurs d'énergie libre calculées par le modèle de données par rapport à des valeurs réelles sont rétropropagés, et les poids de modèle de toutes les couches sont ajustés pour minimiser l'écart, dans lequel après une phase d'entraînement, le modèle de données entraîné est utilisé pour traiter des entrées en temps réel et pour générer une valeur de l'énergie libre de perméation correspondant à des valeurs des entrées en temps réel ;

la génération (212b) d'un vecteur transformé comprenant des informations sur une dépendance non linéaire des composantes du vecteur de contexte, et une couche dense apprend la dépendance non linéaire d'éléments du vecteur de contexte avec l'énergie libre, dans lequel en faisant passer le vecteur de contexte à travers la couche dense, un autre vecteur de même longueur qui incorpore la non-linéarité est obtenu ; et

l'estimation (212c) de l'énergie libre de perméation dans un neurone de sortie en tant que somme pondérée de composantes du vecteur transformé de la couche dense,

dans lequel le modèle de données est déployé pour cribler virtuellement de petites molécules de type médicament, et les un ou plusieurs processeurs matériels sont conçus pour concevoir une solution pour estimer l'énergie libre de perméation de petites molécules à travers des membranes.

2. Système (100), comprenant :

un ou plusieurs processeurs matériels (102) ;
une interface de communication (112) ; et
une mémoire (104) stockant une pluralité d'instructions, dans lequel la pluralité d'instructions, lorsqu'elles sont exécutées, amènent les un ou plusieurs processeurs matériels à :

générer un premier ensemble de caractéristiques sur la base d'une interaction d'une molécule avec une membrane lipidique, en effectuant une pluralité de simulations de dynamique moléculaire, dans lequel des valeurs du premier ensemble de caractéristiques sont collectées en tant que données de série temporelle, dans lequel le premier ensemble de caractéristiques comprend a) une énergie d'interaction de Lennard-Jones, LJ, de la molécule avec la membrane lipidique, b) une aire par lipide de la membrane lipidique, c) un écart quadratique moyen (RMS) d'une pluralité de molécules lipidiques par rapport à une position initiale correspondante, et d) un écart RMS pour une combinaison d'une pluralité de membranes lipidiques et de la molécule ;

générer un second ensemble de caractéristiques sur la base d'une interaction de la molécule avec un solvant en effectuant la pluralité de simulations de dynamique moléculaire, dans lequel des valeurs du second ensemble de caractéristiques sont collectées en tant que données de série temporelle, dans lequel le second ensemble de caractéristiques comprend a) une aire de surface de la molécule à laquelle le solvant peut accéder, b) une enthalpie molécule-solvant, c) une énergie d'interaction LJ entre la molécule et le solvant, et d) une énergie de liaison de la molécule ;

générer un ensemble de données concaténé comprenant des valeurs du premier ensemble de caractéristiques et du second ensemble de caractéristiques, extraites d'une pluralité d'instances des données de série temporelle du premier ensemble de caractéristiques et du second ensemble de caractéristiques, dans lequel la génération du premier ensemble de caractéristiques et du second ensemble de caractéristiques comprend :

la génération (302) d'une molécule dans un système de solvant en spécifiant a) des positions et des vitesses initiales de tous les atomes dans la molécule dans le système de solvant, et b) une pluralité de conditions de simulation de la molécule dans le système de solvant, dans lequel des simulations sont effectuées à température ambiante, 300 K, ou température physiologique, 310 K, pression atmosphérique, pour une application pharmaceutique, dans lequel la molécule dans le système de solvant est générée en créant une boîte de simulation et en insérant la molécule et les molécules de solvant dans la boîte de simulation, dans lequel l'insertion se réfère à la spécification de positions et de vitesses initiales de tous les atomes dans la molécule dans le système de solvant et la molécule est insérée au centre de la boîte de simulation et le volume de boîte restant est rempli de molécules de solvant ;

la génération (304) d'une molécule dans un système de membrane lipidique en spécifiant a) des positions et des vitesses initiales de tous les atomes dans la molécule dans le système de membrane lipidique, et b) une pluralité de conditions de simulation de la molécule dans le système de membrane lipidique, dans lequel la molécule dans le système de membrane lipidique est générée en créant une boîte de simulation, en spécifiant a) des positions et des vitesses initiales de tous les atomes dans la molécule dans le système de membrane lipidique et des molécules lipidiques sont agencées sous la forme d'une membrane de type lipide et la molécule lipidique est insérée au niveau d'un plan médian de membrane,

dans lequel la molécule dans le système de solvant et la molécule dans le système de membrane lipidique sont soumises à une minimisation d'énergie potentielle, et des chevauchements entre une ou plusieurs paires d'atomes sont éliminés ;

la génération (306) d'une trajectoire comprenant la position et les vitesses de tous les atomes dans la molécule dans le système de solvant et la molécule dans le système de membrane lipidique, à tous les pas de temps au cours de la pluralité de simulations dynamiques moléculaires ; et

l'extraction (308) de valeurs du premier ensemble de caractéristiques et du second ensemble de caractéristiques, à partir de la trajectoire, dans lequel, dans la trajectoire, chaque configuration unique est appelée un instantané constitué de positions et de vitesses de toutes les particules à un instant particulier, des valeurs de chacune des caractéristiques formant le premier ensemble de caractéristiques et le second ensemble de caractéristiques sont calculées en fonction des positions et des vitesses des particules, et de multiples instantanés sont extraits de la trajectoire générée et des valeurs de ceux-ci sont calculées ;

le prétraitement de l'ensemble de données concaténé pour générer un ensemble de données prétraité ;

la représentation de l'ensemble de données prétraité dans un format de réseau bidimensionnel (2D) en tant que données de série temporelle avec de multiples caractéristiques ; et

la génération d'une énergie libre de perméation en traitant l'ensemble de données prétraité dans le format de réseau 2D à l'aide d'un modèle de données d'apprentissage automatique, dans lequel le modèle de données d'apprentissage automatique est généré à l'aide de données obtenues pour une pluralité de combinaisons molécule-couche lipidique qui sont des molécules différentes et des couches lipidiques différentes, dans lequel les données sont en outre utilisées pour entraîner le modèle de données comprenant des couches de mémoire à long terme, LSTM, et une couche d'attention d'un réseau neuronal, dans lequel chaque cellule LSTM de la couche LSTM prend un vecteur de caractéristique à chaque pas de temps $(x_t)$, un état caché de la cellule précédente $(h_{t-1})$, et un état de cellule précédent $(c_{t-1})$ en tant qu'entrée, et produit un état de cellule $(c_t)$ et un état caché $(h_t)$ en tant que sortie, dans lequel la couche LSTM est un moyen de codage de l'entrée de sorte que des couches suivantes apprennent des informations à partir des états cachés, par :

la génération d'un vecteur de contexte, en alimentant des états cachés à partir de la couche LSTM dans la couche d'attention, comprenant des informations sur une dépendance au temps, une pertinence de caractéristique en déduisant que divers poids de modèle utilisés lors de la construction du modèle de données sont ajustés pendant un processus d'entraînement de sorte qu'une sortie est sensible à certaines caractéristiques plus que d'autres, et une similarité, pour la molécule et la membrane lipidique dans l'ensemble de données prétraité stocké dans le format de réseau 2D, dans lequel le réseau 2D représente une évolution de la molécule dans le système de solvant et/ou de la molécule dans le système de membrane lipidique avec le temps et en se déplaçant sur des rangées dans le réseau 2D, des informations sur la façon dont les caractéristiques de la molécule dans le système de solvant et/ou de la molécule dans le système de membrane lipidique changent avec le temps sont obtenues en se référant à la dépendance au temps, dans lequel le vecteur de contexte est calculé à l'aide de la couche d'attention du modèle de données, et capture une similarité entre différentes molécules, dans lequel le vecteur de contexte représente une importance relative des caractéristiques pour calculer l'énergie libre de perméation et le vecteur de contexte réduit également des informations séquentielles en un vecteur simple qui est mappé sur une sortie du modèle de données se référant à l'énergie libre de perméation, dans lequel la couche LSTM et la couche d'attention sont utilisées séquentiellement pour apprendre la dépendance au temps, la pertinence de caractéristique, et la similarité entre des molécules, dans lequel pendant l'entraînement, des écarts de valeurs d'énergie libre calculées par le modèle de données par rapport à des valeurs réelles sont rétropropagés, et les poids de modèle de toutes les couches sont ajustés pour minimiser l'écart, dans lequel après une phase d'entraînement, le modèle de données entraîné est utilisé pour traiter des entrées en temps réel et pour générer une valeur de l'énergie libre de perméation correspondant à des valeurs des entrées en temps réel ;

la génération d'un vecteur transformé comprenant des informations sur une dépendance non linéaire des composantes du vecteur de contexte, et une couche dense apprend la dépendance non linéaire d'éléments du vecteur de contexte avec l'énergie libre, dans lequel en faisant passer le vecteur de contexte à travers la couche dense, un autre vecteur de même longueur qui incorpore la non-linéarité est obtenu ; et

l'estimation de l'énergie libre de perméation dans un neurone de sortie en tant que somme pondérée de composantes du vecteur transformé de la couche dense,

dans lequel le modèle de données est déployé pour cribler virtuellement de petites molécules de type médicament, et le système (100) est conçu pour concevoir une solution pour estimer l'énergie libre de perméation de petites molécules à travers des membranes.

**3.** Un ou plusieurs supports de stockage d'informations lisibles par machine non transitoires comprenant une ou plusieurs instructions qui, lorsqu'elles sont exécutées par un ou plusieurs processeurs matériels, amènent :

la génération d'un premier ensemble de caractéristiques sur la base d'une interaction d'une molécule avec une membrane lipidique, en effectuant une pluralité de simulations de dynamique moléculaire, dans lequel des valeurs du premier ensemble de caractéristiques sont collectées en tant que données de série temporelle, dans lequel le premier ensemble de caractéristiques comprend a) une énergie d'interaction de Lennard-Jones, LJ, de la molécule avec la membrane lipidique, b) une aire par lipide de la membrane lipidique, c) un écart quadratique moyen (RMS) d'une pluralité de molécules lipidiques par rapport à une position initiale correspondante, et d) un écart RMS pour une combinaison d'une pluralité de membranes lipidiques et de la molécule ;

la génération d'un second ensemble de caractéristiques sur la base d'une interaction de la molécule avec un solvant en effectuant la pluralité de simulations de dynamique moléculaire, dans lequel des valeurs du second ensemble de caractéristiques sont collectées en tant que données de série temporelle, dans lequel le second ensemble de caractéristiques comprend a) une aire de surface de la molécule à laquelle le solvant peut accéder, b) une enthalpie molécule-solvant, c) une énergie d'interaction LJ entre la molécule et le solvant, et d) une énergie de liaison de la molécule ;

la génération d'un ensemble de données concaténé comprenant des valeurs du premier ensemble de caractéristiques et du second ensemble de caractéristiques, extraites d'une pluralité d'instances des données de série temporelle du premier ensemble de caractéristiques et du second ensemble de caractéristiques, dans lequel la génération du premier ensemble de caractéristiques et du second ensemble de caractéristiques comprend :

la génération (302) d'une molécule dans un système de solvant en spécifiant a) des positions et des vitesses initiales de tous les atomes dans la molécule dans le système de solvant, et b) une pluralité de conditions de simulation de la molécule dans le système de solvant, dans lequel des simulations sont effectuées à température ambiante, 300 K, ou température physiologique, 310 K, pression atmosphérique, pour une application pharmaceutique, dans lequel la molécule dans le système de solvant est générée en créant une boîte de simulation et en insérant la molécule et les molécules de solvant dans la boîte de simulation, dans lequel l'insertion se réfère à la spécification de positions et de vitesses initiales de tous les atomes dans la molécule dans le système de solvant et la molécule est insérée au centre de la boîte de simulation et le volume de boîte restant est rempli de molécules de solvant ;

la génération (304) d'une molécule dans un système de membrane lipidique en spécifiant a) des positions et des vitesses initiales de tous les atomes dans la molécule dans le système de membrane lipidique, et b) une pluralité de conditions de simulation de la molécule dans le système de membrane lipidique, dans lequel la molécule dans le système de membrane lipidique est générée en créant une boîte de simulation, en spécifiant a) des positions et des vitesses initiales de tous les atomes dans la molécule dans le système de membrane lipidique et des molécules lipidiques sont agencées sous la forme d'une membrane de type lipide et la molécule lipidique est insérée au niveau d'un plan médian de membrane,

dans lequel la molécule dans le système de solvant et la molécule dans le système de membrane lipidique sont soumises à une minimisation d'énergie potentielle, et des chevauchements entre une ou plusieurs paires d'atomes sont éliminés ;

la génération (306) d'une trajectoire comprenant la position et les vitesses de tous les atomes dans la molécule dans le système de solvant et la molécule dans le système de membrane lipidique, à tous les pas de temps au cours de la pluralité de simulations dynamiques moléculaires ; et

l'extraction (308) de valeurs du premier ensemble de caractéristiques et du second ensemble de caractéristiques, à partir de la trajectoire, dans lequel, dans la trajectoire, chaque configuration unique est appelée un instantané constitué de positions et de vitesses de toutes les particules à un instant particulier, des valeurs de chacune des caractéristiques formant le premier ensemble de caractéristiques et le second ensemble de caractéristiques sont calculées en fonction des positions et des vitesses des particules, et de multiples instantanés sont extraits de la trajectoire générée et des valeurs de ceux-ci sont calculées ;

le prétraitement de l'ensemble de données concaténé pour générer un ensemble de données prétraité ;

la représentation de l'ensemble de données prétraité dans un format de réseau bidimensionnel (2D) en tant que données de série temporelle avec de multiples caractéristiques ; et

la génération d'une énergie libre de perméation en traitant l'ensemble de données prétraité dans le format de réseau 2D à l'aide d'un modèle de données d'apprentissage automatique, dans lequel le modèle de données d'apprentissage automatique est généré à l'aide de données obtenues pour une pluralité de combinaisons molécule-couche lipidique qui sont des molécules différentes et des couches lipidiques différentes, dans lequel les données sont en outre utilisées pour entraîner le modèle de données comprenant des couches de mémoire à long terme, LSTM, et

une couche d'attention d'un réseau neuronal, dans lequel chaque cellule LSTM de la couche LSTM prend un vecteur de caractéristique à chaque pas de temps ($x_t$), un état caché de la cellule précédente ($h_{t-1}$), et un état de cellule précédent ($c_{t-1}$) en tant qu'entrée, et produit un état de cellule ($c_t$) et un état caché ($h_t$) en tant que sortie, dans lequel la couche LSTM est un moyen de codage de l'entrée de sorte que des couches suivantes apprennent des informations à partir des états cachés, comprenant :

la génération d'un vecteur de contexte, en alimentant des états cachés de la couche LSTM dans la couche d'attention, comprenant des informations sur une dépendance au temps, une pertinence de caractéristique en déduisant que divers poids de modèle utilisés lors de la construction du modèle de données sont ajustés pendant un processus d'entraînement de sorte qu'une sortie est sensible à certaines caractéristiques plus que d'autres, et une similarité, pour la molécule et la membrane lipidique dans l'ensemble de données prétraité stocké dans le format de réseau 2D, dans lequel le réseau 2D représente une évolution de la molécule dans le système de solvant et/ou de la molécule dans le système de membrane lipidique avec le temps et en se déplaçant sur des rangées dans le réseau 2D, des informations sur la façon dont les caractéristiques de la molécule dans le système de solvant et/ou de la molécule dans le système de membrane lipidique changent avec le temps sont obtenues en se référant à la dépendance au temps, dans lequel le vecteur de contexte est calculé à l'aide de la couche d'attention du modèle de données, et capture une similarité entre différentes molécules, dans lequel le vecteur de contexte représente une importance relative des caractéristiques pour calculer l'énergie libre de perméation et le vecteur de contexte réduit également des informations séquentielles en un vecteur simple qui est mappé à la sortie du modèle de données en se référant à l'énergie libre de perméation, dans lequel la couche LSTM et la couche d'attention sont utilisées séquentiellement pour apprendre la dépendance au temps, la pertinence de caractéristique, et la similarité entre des molécules, dans lequel pendant l'entraînement, des écarts de valeurs d'énergie libre calculées par le modèle de données par rapport à des valeurs réelles sont rétropropagés, et les poids de modèle de toutes les couches sont ajustés pour minimiser l'écart, dans lequel après une phase d'entraînement, le modèle de données entraîné est utilisé pour traiter des entrées en temps réel et pour générer une valeur de l'énergie libre de perméation correspondant à des valeurs des entrées en temps réel ;

la génération d'un vecteur transformé comprenant des informations sur une dépendance non linéaire des composantes du vecteur de contexte, et une couche dense apprend la dépendance non linéaire d'éléments du vecteur de contexte avec l'énergie libre, dans lequel en faisant passer le vecteur de contexte à travers la couche dense, un autre vecteur de même longueur qui incorpore la non-linéarité est obtenu ; et

l'estimation de l'énergie libre de perméation dans un neurone de sortie en tant que somme pondérée de composantes du vecteur transformé de la couche dense,

dans lequel le modèle de données est déployé pour cribler virtuellement de petites molécules de type médicament, et un système (100) est conçu pour concevoir une solution pour estimer l'énergie libre de perméation de petites molécules à travers des membranes.

100

FIG. 1

Generating, via one or more hardware processors, a first set of features based on interaction of a molecule with a lipid membrane, by performing a plurality of molecular dynamic simulations, wherein values of the first set of features are collected as a time series data

202

Generating, via the one or more hardware processors, a second set of features based on interaction of the molecule with a solvent, by performing a plurality of molecular dynamic solutions, wherein the values of the second set of features are collected as a time series data

204

Generating, via the one or more hardware processors, a concatenated data set comprising values of the first set of features and the second set of features, extracted from a plurality of instances of the first set of features and the second set of features

206

Pre-processing, via the one or more hardware processors, the concatenated data set to generate a pre-processed data set

208

A

200

FIG. 2A

A

Representing, via the one or more hardware processors, the pre-processed data set in a 2-Dimensional (2D) array format _210

Generating a context vector comprising information on time dependency, feature relevance, and similarity, for the molecule and the lipid membrane in the pre-processed dataset stored in the 2D array format _212a

Generating a transformed vector comprising non-linear dependency of the components of the context vector _212b

Generating free energy of permeation as weighted sum of components of the transformed vector _212c

Generating a predicted free energy of permeation by processing the pre-processed data set in the 2D array format using a machine learning data model

_212

200

FIG. 2B

generating a molecule in solvent system by specifying a) initial positions and velocities of all the atoms in the molecule in solvent system, and b) a plurality of simulating conditions of the molecule in solvent system ⟋302

generating a molecule in lipid membrane system by specifying a) initial positions and velocities of all the atoms in the molecule in lipid membrane system, and b) a plurality of simulating conditions of the molecule in lipid membrane system ⟋304

generating a trajectory comprising the position and velocities of all the atoms in the molecule in solvent system and the  molecule in lipid membrane system, at all timesteps during the course of the plurality of molecular dynamic simulations ⟋306

Extracting values of the first set of features and the second set of features, from the trajectory ⟋308

300 ⟋

FIG. 3

FIG. 4A

FIG. 4B

FIG. 5

FIG. 6

FIG. 7

FIG. 8A

FIG. 8B

FIG. 8C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202221016248

**Non-patent literature cited in the description**

- **BENNETT W. F. DREW et al.** *Predicting Small Molecule Transfer Free Energies by Combining Molecular Dynamics Simulations and Deep Learning*

- **BERISHVILI VLADIMIR P. et al.** *Time-Domain Analysis of Molecular Dynamics Trajectories Using Deep Neural Networks: Application to Activity Ranking of Tankyrase Inhibitors*
- **TSAI SUN-TING et al.** *Learning molecular dynamics with simple language model built upon long short-term memory neural network*